# EUROPEAN PATENT APPLICATION

(11) **EP 4 818 857 A1**
(43) Date of publication of application: **07.10.2026**
(21) Application number: 26170085.0
(22) Date of filing: 31.03.2026
(51) Int. Cl.: G06F 3/01, A61F 4/00

(54) **METHODS FOR OPERATING A BRAIN COMPUTER INTERFACE (BCI) SYSTEM IN SAFE MODE**

(30) Priority: 31.03.2025 US 202563781214 P; 26.03.2026 US 202619630292
(71) Applicant: Echo Neurotechnologies Corporation, San Francisco, CA 94107 (US)
(72) Inventor: BILDERSEE, Adam Schembri, SAN FRANCISCO, 94107 (US); MOSES, David Aaron, SAN FRANCISCO, 94107 (US)
(74) Representative: Eisenführ Speiser

(57) **Abstract**

Some embodiments are directed to systems and methods for operating a brain computer interface (BCI) system in safe mode. In one aspect, a computing device, while operating in a first mode, decodes a set of signals that are received from a brain of a human subject. The computing device determines a first set of intended actions of the human subject based on the decoding. The first set of intended actions includes an intended motor action or an intended verbal action for controlling the computing device. The computing device receives an instruction to operate the computing device in a second mode. The computing device, in accordance with receiving the instruction, operates the computing device in the second mode. The second mode limits operations of the computing device to a subset of operations, less than all operations that the computing device is configured to perform in the first mode.

## Description

### RELATED APPLICATIONS

This application is a continuation of U.S. Patent Application No. 19/630,292, filed March 26, 2026, which claims priority to U.S. Provisional Application No. 63/781,214, filed March 31, 2025, each of which is incorporated by reference herein in its entirety.

### TECHNICAL FIELD

The disclosed embodiments relate generally to brain computer interface (BCI) systems, and more specifically to systems, methods, and user interfaces for operating a BCI system in safe mode.

### BACKGROUND

Neurological conditions can result in the loss of communication and autonomy. Brain computer interface (BCI) technologies can assist individuals with neurological impairments, by recording and processing brain signals and translating the signals into outputs.

### SUMMARY

The present disclosure describes, amongst others, methods and user interfaces for operating a BCI system in safe mode. BCI system can apply machine learning algorithms to decode brain signals of a user (e.g., patient or human subject) to determine one or more intended actions of the user. In some circumstances, the algorithms employed in a BCI system may produce erroneous or inaccurate predictions of user intent. For example, poor BCI performance may occur when the algorithms have not been fully calibrated, or when recalibration is necessary because algorithm performance has degraded.

As disclosed, in some situations where the algorithms employed in a BCI system produce erroneous or inaccurate predictions of user intent, a computing device may transition from a normal mode of operation to a safe mode of operation. Safe mode improves reliability of BCI operation under degraded neural-signal conditions, reduces unintended machine actions, and preserves a usable control path between the user and the computer. In some embodiments, in the safe mode of operation, partial control of the computing device may be possible through a simplified user interface, which reconfigures decoding by disabling higher-dimensional or lower-reliability outputs and constraining the action space to commands that are more robustly decodable. In some embodiments, the computing device limits output of the intended actions of the user to a cursor control when operating in the safe mode. In some embodiments, the computing device limits output of the intended actions of the user to cursor control in limited directions (e.g., up, down, left or right directions) when operating in the safe mode.

As disclosed, operating the computing device in safe mode prioritizes patient safety by providing a fallback mechanism when the normal mode of operation is compromised. Operating in safe mode ensures that the computing device does not deliver unexpected or inappropriate results and minimizes potential harm or frustration to the user while allowing the user to retain partial control of the computing device. As disclosed, in some embodiments, user inputs to the computing device in the safe mode can provide reinforcement learning input to the computing device to work toward bootstrapping the full system back up. As disclosed, in some embodiments, operating in safe mode reduces system flexibility in exchange for increased system reliability.

In accordance with some embodiments, a method is performed at a computing device that includes a display, one or more processors, and memory. The method includes, while operating in a first mode (e.g., normal mode), decoding a set of signals that are received from a brain of a human subject. The method includes determining a first set of one or more intended actions of the human subject based on the decoding. The first set of intended actions includes an intended motor action, an intended cursor action, or an intended verbal action for controlling the computing device. The method includes receiving a first instruction to operate the computing device in a second mode (e.g., safe mode), different from the first mode. The method includes, in accordance with receiving the first instruction, operating the computing device in the second mode. The second mode limits operations of the computing device to a subset of operations, less than all of a plurality of operations that the computing device is configured to perform in the first mode.

In some embodiments, the set of signals comprises a signal associated with an attempt by the human subject to speak. In some embodiments, the set of signals comprises a signal associated with an attempt by the human subject to move a body part of the human subject. In some embodiments, the set of signals comprises a signal associated with an attempt by the human subject to control a facial expression of the human subject. In some embodiments, the set of signals comprises a signal associated with an attempt by the human subject to control an external device.

In some embodiments, determining the first set of intended actions of the human subject based on the decoding includes, for a respective determined intended action: determining a respective error score corresponding to the respective determined intended action; and in accordance with a determination that the respective error score satisfies a threshold value, generating the first instruction to operate the computing device in the second mode.

In some embodiments, operating the computing device in the second mode includes generating and displaying a user interface that includes a set of (e.g., one or more) user-selectable input controls. The set of user-selectable input controls corresponds to the subset of operations that the computing device is configured to perform in the second mode.

In accordance with some embodiments, a computing device includes a display, one or more processors, and memory coupled to the one or more processors. The memory stores one or more programs configured for execution by the one or more processors. The one or more programs include instructions for performing any of the methods disclosed herein.

In accordance with some embodiments, a computer system includes one or more processors, and memory coupled to the one or more processors. The memory stores one or more programs configured for execution by the one or more processors. The one or more programs include instructions for performing any of the methods disclosed herein.

In accordance with some embodiments, a non-transitory computer readable storage medium stores one or more programs configured for execution by a computing device having a display, one or more processors, and memory. The one or more programs include instructions for performing any of the methods disclosed herein.

In accordance with some embodiments, a non-transitory computer readable storage medium stores one or more programs configured for execution by a computer system having one or more processors, and memory. The one or more programs include instructions for performing any of the methods disclosed herein.

Thus methods, systems, and graphical user interfaces are disclosed that operate a brain user interface computing device in safe mode.

Note that the various embodiments described above can be combined with any other embodiments described herein. The features and advantages described in the specification are not all inclusive and, in particular, many additional features and advantages will be apparent to one of ordinary skill in the art in view of the drawings, specification, and claims. Moreover, it should be noted that the language used in the specification has been principally selected for readability and instructional purposes and may not have been selected to delineate or circumscribe the inventive subject matter.

### BRIEF DESCRIPTION OF THE DRAWINGS

For a better understanding of the aforementioned systems, methods, and graphical user interfaces, reference should be made to the Detailed Description of Embodiments below, in conjunction with the following drawings in which like reference numerals refer to corresponding parts throughout the figures. The accompanying drawings, which are included to provide a further understanding of the embodiments, are incorporated herein, constitute a part of the specification, illustrate the described embodiments, and, together with the description, serve to explain the underlying principles.
Figure 1A illustrates an example operating environment, in accordance with some embodiments.
Figure 1B illustrates example operating modes of a computing device, in accordance with some embodiments.
Figure 1C is a graph showing an actual time-series dataset of neural activity of a patient, in accordance with some embodiments.
Figure 1D is a graph showing neural features extracted from the neural activity dataset of Figure 1C, in accordance with some embodiments.
Figures 2A, 2B, and 2C illustrate an example implant device, in accordance with some embodiments.
Figure 2D is a block diagram showing an example data path, control path, and power path of a implant device, in accordance with some embodiments.
Figure 3A illustrates an example wearable device, in accordance with some embodiments.
Figure 3B is a block diagram showing an example data path, control path, and power path of a wearable device, in accordance with some embodiments.
Figure 4 is a block diagram illustrating an example computing device in accordance with some embodiments.
Figure 5 is a block diagram illustrating an example server system in accordance with some embodiments.
Figure 6A illustrates a workflow corresponding to a normal mode of operation of a computing device, in accordance with some embodiments.
Figure 6B illustrates a workflow corresponding to a safe mode of operation of a computing device, in accordance with some embodiments.
Figures 7A to 7F illustrate a safe mode user interface, in accordance with some embodiments.
Figure 8 is a flowchart of an example process for operating a computing device in safe mode, in accordance with some embodiments.

Like reference numerals refer to corresponding parts throughout the several views of the drawings.

### DETAILED DESCRIPTION OF EMBODIMENTS

Reference will now be made in detail to specific embodiments, examples of which are illustrated in the accompanying drawings. In the following detailed description, numerous non-limiting specific details are set forth in order to assist in understanding the subject matter presented herein. But it will be apparent to one of ordinary skill in the art that various alternatives may be used without departing from the scope of the claims and the subject matter may be practiced without these specific details. For example, it will be apparent to one of ordinary skill in the art that the subject matter presented herein can be implemented on many types of electronic devices with digital video capabilities.

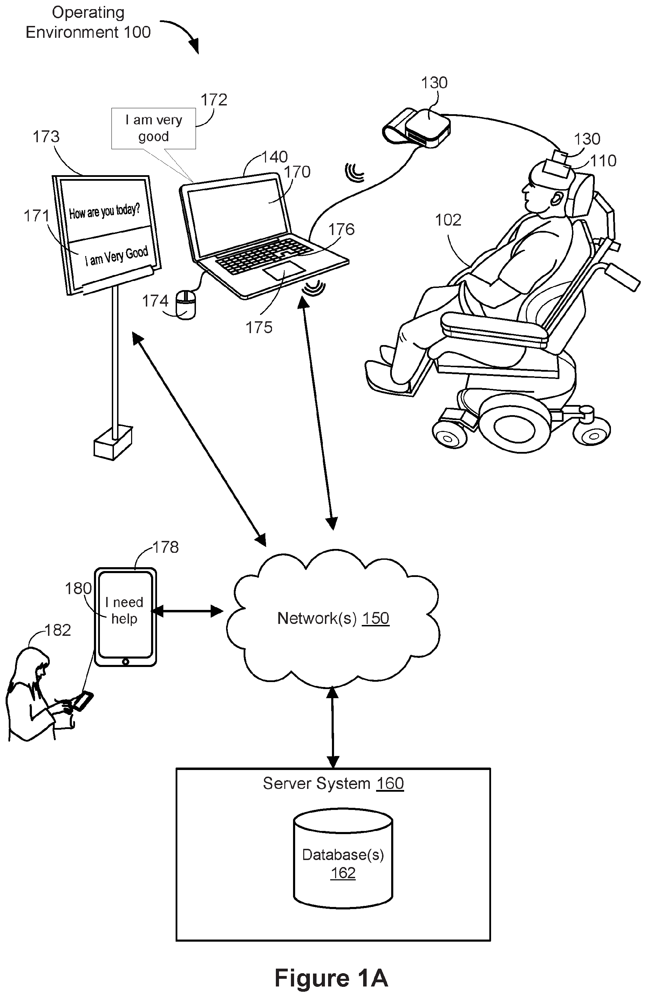

Figure 1A illustrates an example operating environment 100 of a brain computer interface (BCI) system, in accordance with some embodiments.

As used herein, a BCI system is a system that acquires brain signals from a user and determines, from the signals, into one or more intended actions (e.g., functional intents) of the user. In some embodiments, the BCI system generates and executes commands for controlling one or more external devices without requiring traditional muscle movements of the user. Because no user movement or vocalization is required to control the BCI system, the BCI system can assist users with disabilities, thereby enhancing the user's capabilities and improving their quality of life.

The operating environment 100 depicts a human subject 102. As used herein, human subject 102 is also referred to as a "patient" or a "user." In some instances, the human subject 102 is unable to speak or unable to move their limbs. In some embodiments, the BCI system includes an implant device 110 (also referred to herein as a cranial implant device), which is implanted into the skull of the human subject 102. The implant device 110 includes an array of high-density electrode sensors for recording neural signals (e.g., electrical signals) corresponding to motor cortex activity directly from the brain surface of the human subject 102. In some embodiments, the array is a brain-penetrating electrode array or an electrocorticography (ECoG) electrode array. In some embodiments, the electrodes are depth electrodes or surface electrodes. In some embodiments, the recorded neural signals represent specific activities of the brain, including attempted speech, attempted movement, hearing, and/or vision. In some embodiments, the array of electrode sensors are placed on the cortical surface of the brain, either epidurally or subdurally, to record signals directly from the brain. In some embodiments, the array of electrode sensors can penetrate under the scalp.

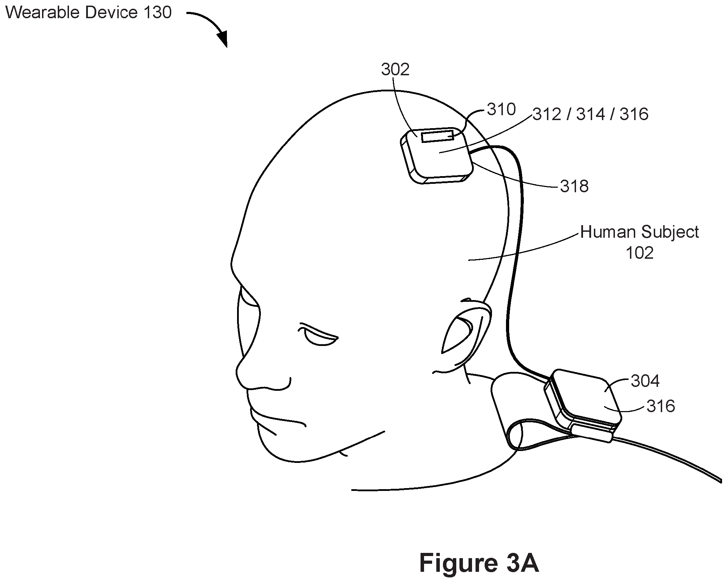

In some embodiments, the BCI system includes a wearable device 130. As described in further detail in Figure 3A, in some embodiments the wearable device 130 includes a patch component that is magnetically and communicatively coupled to the implant device 110. The patch component is configured to transmit power wirelessly into the implant device 110 and receive neural signal data from the implant device 110. In some embodiments, the wearable device 130 includes a relay component that includes electronics for supporting the functionalities of the patch component.

In some embodiments, the BCI system includes a computing device 140 that is communicatively connected with the wearable device 130. The computing device 140 can include a display 170, a keyboard 176 and a pointing device such as a mouse 174 or a touchpad 175. In accordance with some embodiments, the computing device 140 functions as the main interface for the human subject 102. For example, the human subject 102 can access one or more applications executing on the computing device 140 for their daily tasks and activities such as checking email and browsing the web. In some embodiments, the one or more applications executing on the computing device 140 includes a BCI application (e.g., application 440) that is configured to decode the neural signals and interpret the decoded signals to determine one or more intended actions (e.g., functional intents) of the human subject 102. In some embodiments, the BCI application applies one or more machine learning models that are stored locally on the computing device 140 to perform the decoding and interpreting. In some embodiments, the BCI application is configured to execute on the computing device 140 even while the computing device 140 is offline (although an internet connection will be required to receive new models from the server system 160).

In some embodiments, the BCI system includes a server system 160 that is communicatively coupled to the computing device 140 through one or more communication networks 150. The server system 160 includes an operational pipeline that is configured to automatically generate different machine learning models based on respective input data, train the models to optimize their performance on specific tasks, and deploy the models locally on respective computing devices 140. In some embodiments, the server system 160 includes one or more databases 162 for storing data such as raw data obtained from implanted devices corresponding to respective patients, feature data, analytics data, application information, and firmware versions.

With continued reference to Figure 1A, in some embodiments, the BCI system is configured to control one or more output devices to carry out one or more intended actions of the human subject 102 according to the decoded signals. As one example, in some embodiments, the BCI system (e.g., computing device 140 or server system 160) is configured to control a keyboard (e.g., physical keyboard 176 or a virtual keyboard) to generate language and/or numeral outputs according to the decoded signals (e.g., by typing letters, numbers, words and/or sentences using the keyboard), without requiring any voice command and/or limb movement by the human subject 102. As another example, in some embodiments, the BCI system is configured to control a cursor (e.g., by controlling a pointing device such as mouse 174, touchpad 175, or the cursor) according to the decoded signals, without requiring any voice command and/or limb movement by the human subject 102. As another example, in some embodiments, the BCI system is configured to control a display device (e.g., display 170 or external display device 173) to display a visual output (e.g., text entry 171) corresponding to a response from the human subject 102, without requiring any voice command and/or limb movement by the human subject 102. As another example, in some embodiments, the BCI system is configured to translate a visual/text output into a verbal expression and generate an audio output 172 articulating the verbal expression using an audio output component of the computing device 140, without requiring any voice command and/or limb movement by the human subject 102. As another example, in some embodiments, the BCI system is configured to control one or more interfaces of the computing device 140 to navigate through menus and/or switch between tabs of one or more applications executing on the computing device 140, without requiring any voice command and/or limb movement by the human subject 102. As yet another example, in some embodiments, the BCI system is configured to generate an alert according to the decoded signals and transmit the alert to an electronic device 178 that is associated with a caregiver 182 of the human subject 102, for display (e.g., as alert message 180) on a display screen of the electronic device 178.

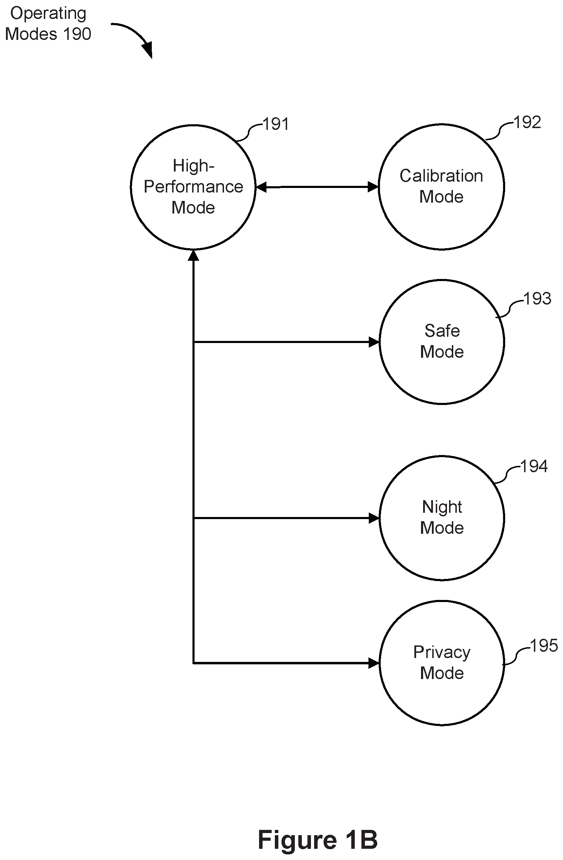

Figure 1B illustrates example operating modes 190 of a computing device 140, in accordance with some embodiments.

In accordance with some embodiments, the computing device 140 can switch between various operating modes. A respective operating mode may have its associated (e.g., distinct) user interface. A human subject 102 may interact with the computing device 140 in specific ways, depending on the operating mode.

Referring to Figure 1B, in some embodiments, the operating modes include a high performance mode 191 that allows a human subject 102 to fully interact with the computing device 140. For example, in the high performance mode 191, the human subject 102 has access to all functionalities of applications that are executing on the computing device. In some embodiments, the high performance mode 191 is also referred as the "normal" mode. In some embodiments, high performance mode 191 is the default operating mode of the computing device.

In some embodiments, the operating modes include a calibration mode 192, which is a sandbox mode used during training tasks.

In some embodiments, the operating modes include a safe mode 193. In some embodiments, the safe mode 193 is a fallback mode for when the high performance mode 191 is not available.

In some embodiments, the operating modes include a night mode 194. When operating in the night mode, the display screen 170 of the computing device 140 is not in use and the computing device 140 has limited functionality due to the screen not being in use.

In some embodiments, the operating modes include a privacy mode 195, where brain signals are not recorded and not decoded.

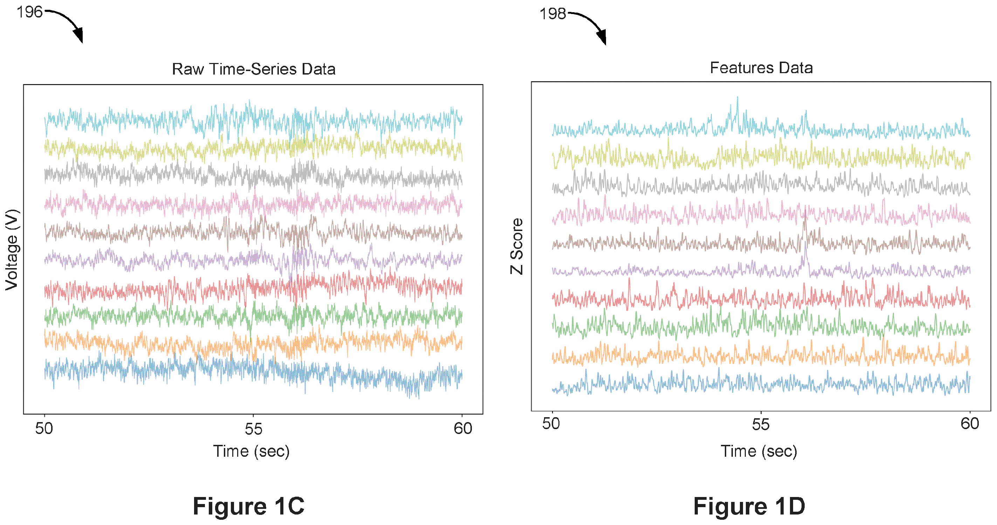

Figure 1C is a graph 196 showing an actual raw time-series dataset of neural activity of a patient collected by an electrode array, in accordance with some embodiments. The dataset is collected by placing electrodes on the surface of the patient's brain. Each of the line plots in the graph 196 corresponds to electrical field signals collected by a respective channel (e.g., an electrode) of the array. The line plots on the graph 196 have been offset for clarity.

Figure 1D is a graph 198 showing a neural-feature time-series dataset extracted from the neural activity data of Figure 1C, in accordance with some embodiments. In some embodiments, the neural-feature time series dataset is generated using a feature processing pipeline that is executed by the computing device 140 (e.g., application 440) or the server system 160 (e.g., operational pipeline 524). The neural features correspond to representations of brain signals that contain information related to underlying neural processing, including neural representations associated with attempted speech and/or motor movements. The values on the y-axis of the graph 198 are z-score values, which are normalized values that indicate how many standard deviations a data point is from the mean of the dataset (computed individually for each feature time series).

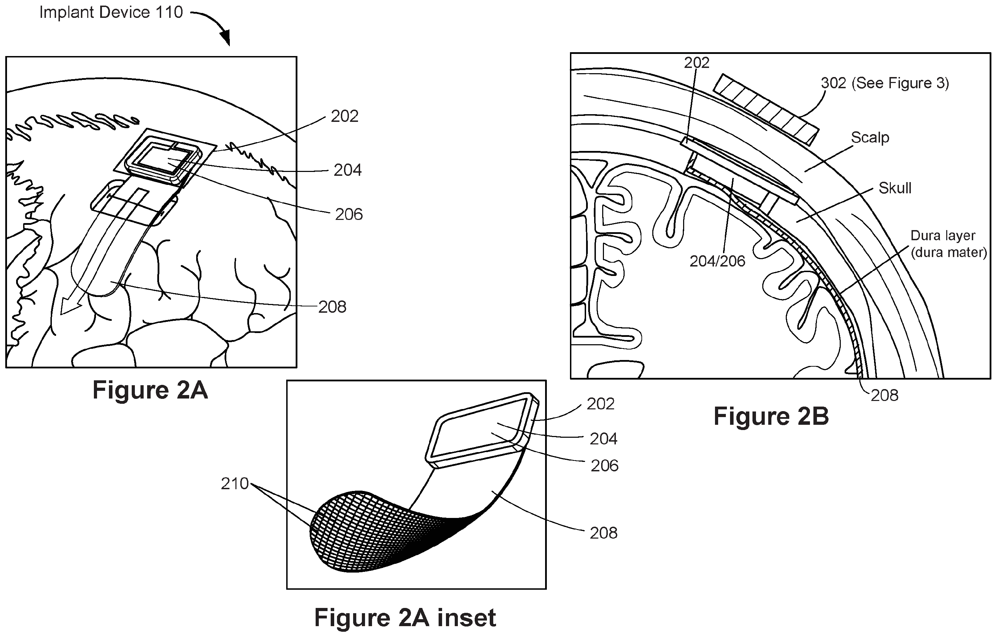

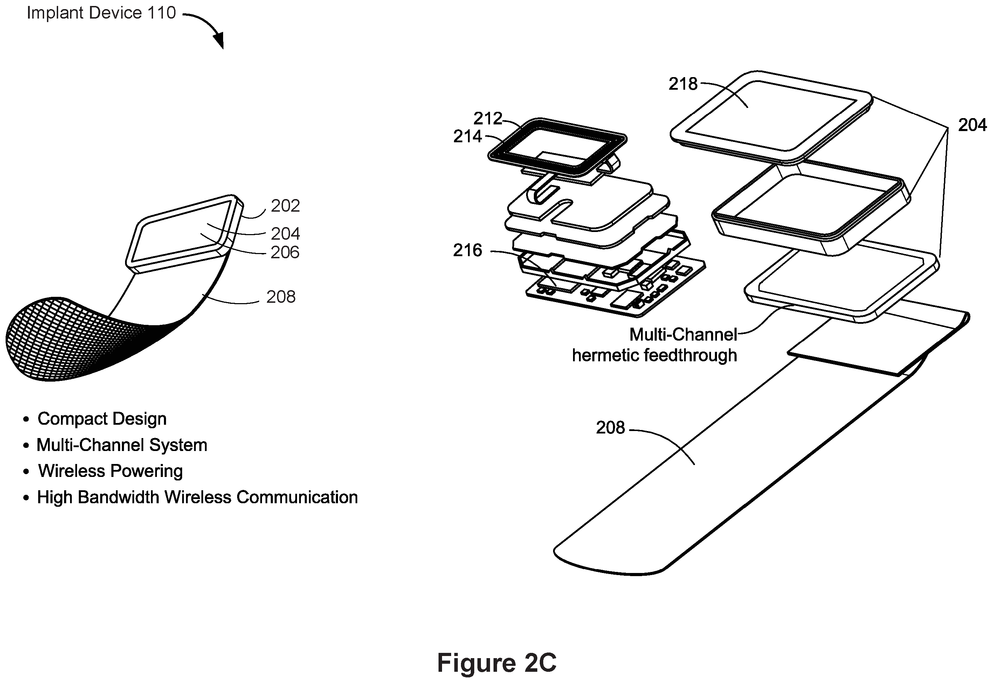

Figures 2A, 2B, and 2C illustrate various views of the implant device 110, in accordance with some embodiments.

In some embodiments, the implant device 110 includes a frame 202, a hermetic package 204 (e.g., hermetically-sealed package), implant electronics 206 (e.g., one or more electronic components) enclosed in the hermetic package 204, and an electrode array 208. As used herein, the electrode array 208 is also referred to as "an array of electrodes" or "an array of electrode sensors." Figure 2A inset shows that the electrode array 208 comprises a thin, flexible array (e.g., with a thickness of around 50 µm to 200 µm) that includes multiple electrode sensors 210. In some embodiments, the electrode array 208 comprises an electrocorticography (ECoG) array that is surgically implanted on the brain's surface.

In some embodiments, the electrode array 208 includes at least 100 electrodes, at least 250 electrodes, at least 500 electrodes, at least 750 electrodes, at least 1000 electrodes, at least 1500 electrodes, at least 2000 electrodes, at least 2500 electrodes, at least 3000 electrodes, at least 4000 electrodes, at least 5000 electrodes, at least 7500 electrodes, or at least 10,000 electrodes. In some embodiments, the electrode array 208 is designed to be placed over a substantial portion of or the entire sensorimotor cortex region, to collect electrophysiologic data with information content of intended speech and movement of a human subject. In some embodiments, the electrode array is designed to be placed over one or more subregions or regions of the cortex, such as the precentral gyrus, postcentral gyrus, posterior superior frontal gyrus, posterior middle frontal gyrus, posterior inferior frontal gyrus region, superior temporal gyrus, or a combination of any of the foregoing. Typically, each electrode 210 of the electrode array 208 corresponds to a channel and is configured to pick up electrical activity from the cortex. A denser electrode array with higher channel count can capture more signals with higher fidelity, which can be used to decode intended actions (e.g., intended speech actions and non-speech actions) of the human subject.

Figure 2B illustrates a coronal cross-sectional view of a brain of a human subject. In some embodiments, the frame 202 is configured to be anchored to a skull of a human subject 102 via a surgical procedure called craniectomy, in which a scalp incision is made and a full-thickness section of bone is removed from the skull. In the example of Figure 2B, the frame 202, the hermetic package 204, and the implant electronics 206 are configured to be positioned above the dural layer (e.g., closer to the scalp), whereas the electrode array 208 is inserted into the sub-dural layer (e.g., underneath the dura mater) of the brain of the human subject 102. During device operation, the electrode array 208 is configured to acquire (e.g., record) motor cortex neural signals (e.g., electrical signals) from the brain of the human subject.

In some embodiments, the implant device 110 includes a battery. In some embodiments, the implant device 110 does not include a battery. In some embodiments, the implant device 110 is configured to receive power signals from a patch component 302 of the wearable device 130. In some embodiments, the implant electronics 206 include a wireless powering coil 212 that is configured to receive electrical power wirelessly from the patch component 302. In some embodiments, the hermetic package 204 includes a cover 218 that is composed of a ceramic material or other dielectric material to facilitate the receiving of power signals via inductive coupling or capacitive coupling. In some embodiments, the wireless powering coil 212 is configured to receive constant electrical power from the patch component 302.

Figure 2B illustrates coupling between the implant device 110 and the patch component 302. In some embodiments, the frame 202 includes one or more magnets that are configured to magnetically couple to one or more complementary magnets positioned at the patch component 302.

In some embodiments, the implant electronics 206 include a wireless communication antenna 214 that is configured to establish a direct wireless link between the implant device 110 and the wearable device 130. In some embodiments, the wireless communication antenna 214 is configured to implement high bandwidth wireless communication to transmit the multiple channels (e.g., hundreds or thousands of channels) of brain signals (e.g., electrical signals) that are collected by the electrode array 208 to the wearable device 130. In some embodiments, the implant device 110 is configured to amplify, digitize, and transmit the signals to the patch component 302 as data packets without interpreting and/or further processing the signals.

In some embodiments, the implant electronics 206 include processing circuitry, communication circuitry, and power circuitry. In some embodiments, the various circuitries are implemented as a multi-channel application-specific integrated circuit (ASIC) chip 216 that enables custom programming of specific tasks. In some embodiments, the ASIC chip 216 is implemented as a system-on-chip (SOC). In some embodiments, the ASIC chip 216 and includes one or more microprocessors and one or more memory blocks such as ROM, RAM, EEPROM, flash memory, or other building blocks.

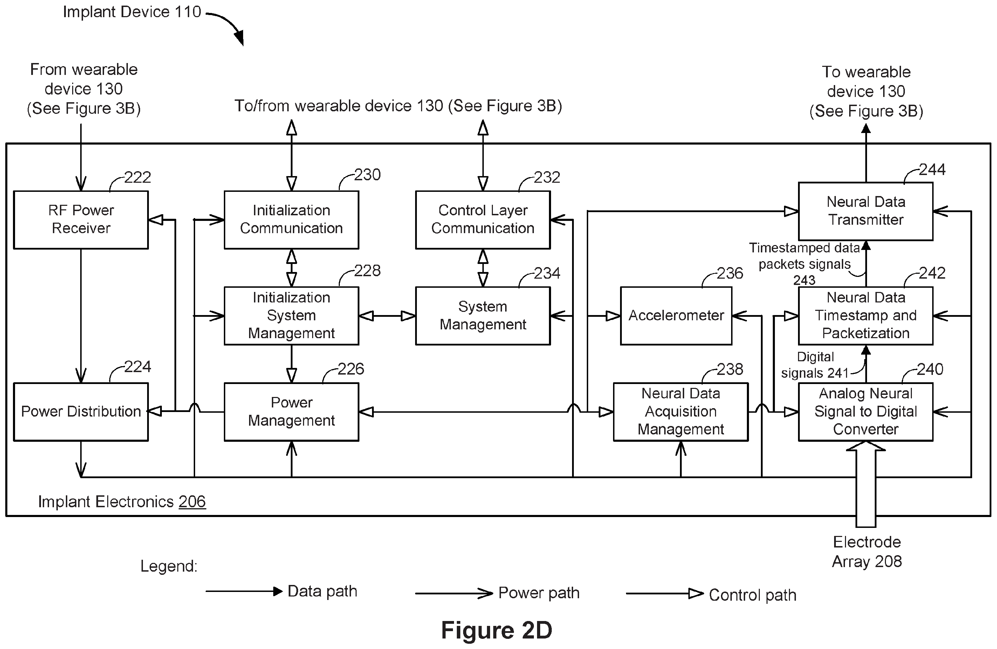

Figure 2D is a block diagram showing an example data path (represented by closed triangle arrows), power path (represented by two-edged arrows), and control path (represented by open triangle arrows) of an implant device 110, in accordance with some embodiments.

The data path of the implant device 110 includes receiving neural signals from the electrode array 208. In some embodiments, the implant device 110 includes an analog neural signal to digital converter module 240 (e.g., an analog to digital converter) for converting the neural signals (e.g., electrical signals) from analog form to digital form (e.g., digital signals 241). The implant device 110 includes a neural data timestamp and packetization module 242 for embedding date/time information to the digital signals and breaking down the digital signals into data packets (e.g., data segments) of smaller data sizes (e.g., timestamped data packets 243). The implant device 110 includes a neural data transmitter module 244 for transmitting the timestamped data packets 243 to the wearable device 130.

In accordance with some embodiments, the implant device 110 does not include a battery (i.e., the implant device 110 is not a battery-operated device). In some embodiments, the implant device 110 receives its power supply from the wearable device 130. In some embodiments, the implant device 110 includes an RF power receiver module 222 for receiving power (e.g., DC power) from the wearable device 130 (e.g., from RF power transmitter module 324 of the wearable device 130) and sending the received power to a power distribution module 224. The power distribution module 224 is configured to distribute the power to the various modules of the implant device 110, including the analog neural signal to digital converter module 240, the neural data timestamp and packetization module 242, the neural data transmitter module 244, a power management module 226, an initialization system management module 228, an initialization communication module 230, a control layer communication module 232, a system management module 234, an accelerometer module 236, and a neural data acquisition module 238.

In some embodiments, the power management module 226 is configured to actively control and monitor the flow of electrical power within the implant device 110. For example, the power management module 226 can regulate voltage, manage current draw, and protect modules such as the neural data acquisition module 238, the analog neural signal to digital converter module 240, the neural data timestamp and packetization module 242, the neural data transmitter module 244, and the accelerometer module 236 against overloads, thus ensuring efficient and safe power distribution to modules or components of the implant device 110.

In some embodiments, the initialization system management module 228 is configured to handle the initialization process when the implant device 110 first starts up, by coordinating the loading and configuration of settings and modules, such as the initialization communication module 230, the system management module 234, and the power management module 226.

In some embodiments, the initialization communication module 230 is configured to set up and prepare the communication channels and protocols needed for the implant device 110 to interact with the wearable device 130 at the beginning of its operation.

In some embodiments, the control layer communication module 232 is configured to manage the flow of data between different modules of the implant device 110 and between the implant device 110 and the wearable device 130.

In some embodiments, the system management module 234 is configured to manage and monitor the performance, health, and operation of the implant device 110. For example, the system management module 234 can track system parameters such as temperature, voltage, and power consumption to ensure stable operation of the implant device 110. In some instances, the system management module 234 can identify system failures, log errors, and provide alerts for troubleshooting.

In some embodiments, the accelerometer module 236 (e.g., hardware accelerator) is specifically designed to significantly speed up the execution of specific tasks by offloading them from the main processor of the ASIC chip 216.

In some embodiments, the neural data acquisition module 238 is configured to record electrical activity from the brain (e.g., neurons) using electrodes 210 of the electrode array 208. In some embodiments, the neural data acquisition module 238 is configured to acquire the electrical activity without performing additional processing on the recorded data. In some embodiments, the neural data acquisition module 238 is configured to boost weak neural signals. In some embodiments, the neural data acquisition module 238 is configured to filter out noise and unwanted frequencies to improve signal quality.

Figure 3A illustrates a wearable device 130, in accordance with some embodiments. In some embodiments, the wearable device 130 includes a patch component 302, a relay component 304, and wearable electronics 316 (e.g., electronic components).

In some embodiments, the patch component 302 includes one or more magnets 310 that are configured to magnetically couple and align the patch component 302 to the implant device 110. In some embodiments, the patch component 302 includes one or more coils 312 and/or antenna 314 for transmitting power wirelessly into the implant device 110, transmitting command signals into the implant device 110, and/or receiving neural signal data transmitted from the implant device 110. In some embodiments, the patch component 302 includes wearable electronics 316 (e.g., electronic components) (e.g., to power the coils 312 and/or the antennas 314. In some embodiments, the patch component 302 includes a mechanical housing 318 for holding the magnets 310 the coils 312, the antenna 314, and/or the wearable electronics components 316.

In some embodiments, the relay component 304 includes additional electronics for supporting functionalities of the patch component 302, and for enabling data and power transfer between the patch component 302 and the computing device 140. In some embodiments, the relay component 304 is configured to anchor the wearable device 130 on or near the human subject 102, such as on clothing worn by the human subject, or on a wheelchair or mobility device of the human subject, or on a patient bed of the human subject.

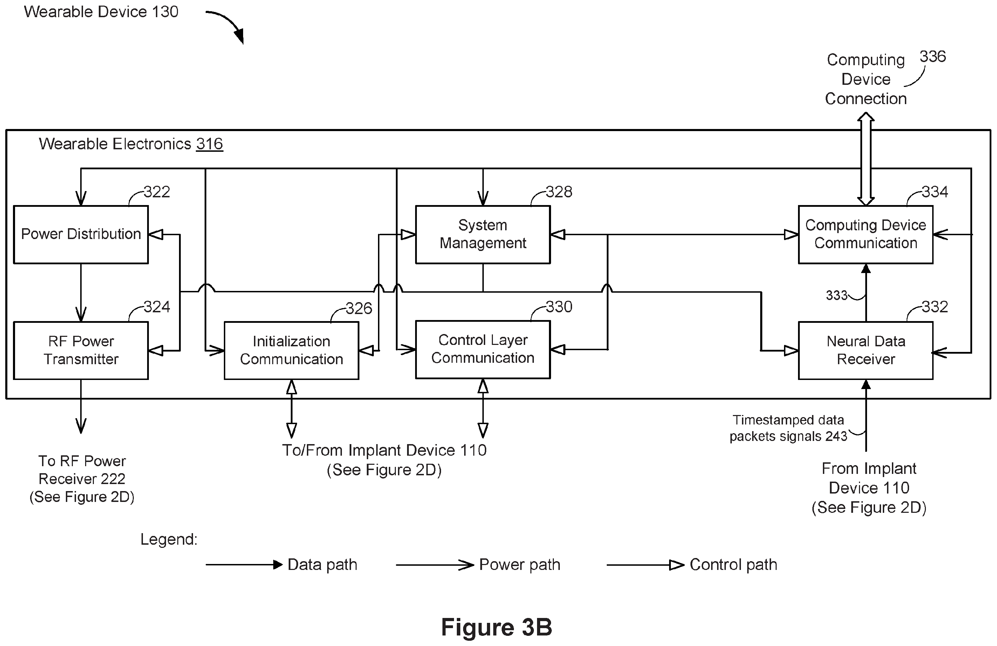

Figure 3B is a block diagram showing an example data path (represented by closed triangle arrows), power path (represented by two-edged arrows), and control path (represented by open triangle arrows) of a wearable device 130, in accordance with some embodiments.

Data path-wise, in some embodiments, the wearable device 130 includes a neural data receiver module 332 for receiving neural signals in the form of timestamped data packets 243 from the implant device 110. In some embodiments, the neural data receiver module 332 generates packetized combined data 333 by further packetizing the timestamped data packets 243 along with data from the wearable device 130 and control commands. The neural data receiver module 332 transmits the packetized combined data 333 to a computing device communication module 334 that is configured to transmit the packetized combined data 333 to the computing device 140 via a connection 336 of the computing device.

The wearable device 130 is connected to an electrical power source. For example, the wearable device 130 can be connected to an electrical power outlet or can include an energy storage device and/or a battery that is configured to supply power to the wearable device 130. In some embodiments, the wearable device 130 includes a RF transmitter module 324 that is configured to transmit electrical power to the implant device 110 (e.g., to the RF power distribution module 322). Ad depicted in Figure 3B, the electrical power source of the wearable device 130 is configured to provide power to various modules of the wearable electronics 316, including an initialization communication module 326, a system management module 328, a control layer communication module 330, the neural data receiver module 332, and the computing device communication module 334.

In some embodiments, the initialization communication module 326 is configured to set up and prepare the communication channels and protocols needed for the wearable device 130 to interact with the implant device 110 at the beginning of its operation.

In some embodiments, the system management module 328 is configured to manage and monitor the performance, health, and operation of the wearable device 130. For example, the system management module 328 can track system parameters such as temperature, voltage, and power consumption to ensure stable operation of the wearable device 130. In some instances, the system management module 328 can identify system failures, log errors, and provide alerts for troubleshooting.

In some embodiments, the control layer communication module 330 is configured to manage the flow of data between different modules of the wearable device 130 and between the wearable device 130 and the implant device 110.

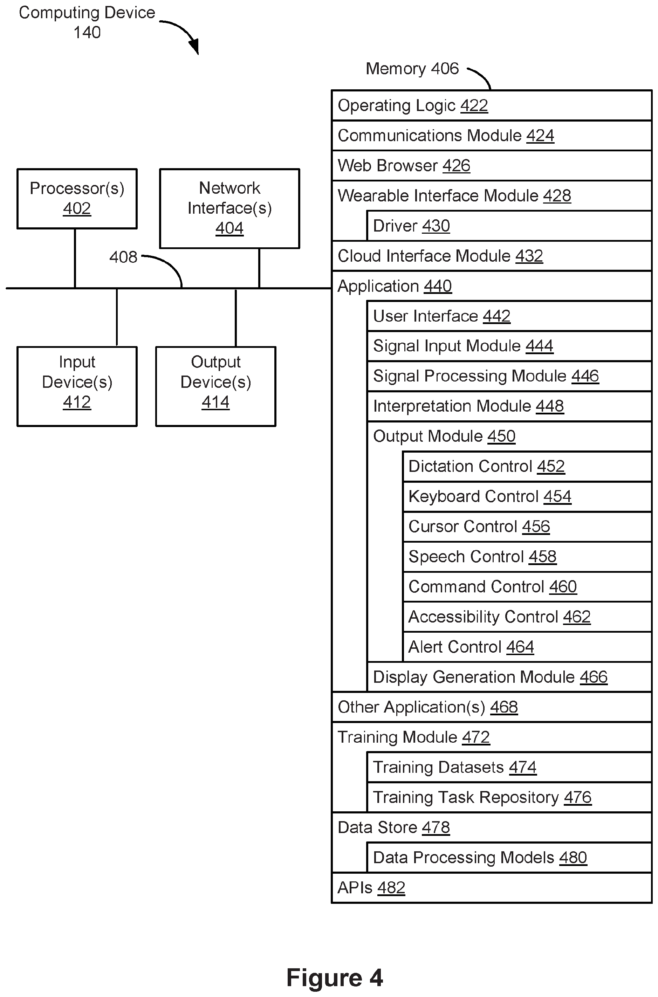

Figure 4 is a block diagram illustrating a computing device 140, in accordance with some embodiments. Various examples of the computing device 140 include a desktop computer, a laptop computer, a tablet computer, a virtual reality (VR) device, an augmented reality (AR) device, or a spatial computing device that blends digital content with the physical world. The computing device 140 includes one or more processors 402 (e.g., processing units (CPUs) or cores), one or more network or communication interfaces 404, memory 406, and one or more communication buses 408 for interconnecting these components. In some embodiments, the communication buses 408 include circuitry (sometimes called a chipset) that interconnects and controls communications between system components. In some embodiments, the computing device 140 includes a user interface 410. The user interface 410 typically includes a display (e.g., display device) 412.

In some embodiments, the computing device 140 includes one or more input devices 412, which facilitate user input, such as a keyboard, a mouse, a voice-command input unit or microphone, a display 170 (e.g., which can be a touch-sensitive display), a touch-sensitive input pad, a gesture capturing camera, or other input buttons or controls. In some embodiments, the computing device 140 uses a microphone and voice recognition or a camera and gesture recognition to supplement or replace the keyboard. In some implementations, the computing device 140 includes one or more cameras, scanners, or photo sensor units for capturing images. In some embodiments, the computing device 140 includes one or more output devices 414, which enable presentation of user interfaces and display content, including one or more speakers and/or one or more visual displays.

The memory 406 includes high-speed random-access memory, such as DRAM, SRAM, DDR RAM, or other random-access solid-state memory devices. In some embodiments, the memory 20406 6 includes non-volatile memory, such as one or more magnetic disk storage devices, one or more optical disk storage devices, one or more flash memory devices, or one or more other non-volatile solid state storage devices. In some embodiments, the memory 406 includes one or more storage devices remotely located from the processors 402. The memory 406, or alternatively the non-volatile memory devices within the memory 406, includes a non-transitory computer-readable storage medium. In some embodiments, the memory 406, or the computer-readable storage medium of the memory 406, stores the following programs, modules, and data structures, or a subset or superset thereof:
- an operating system 422, which includes procedures for handling various basic system services and for performing hardware dependent tasks;
- a communications module 424, which connects the computing device 140 to other devices (e.g., various servers in the server system 160, implant device 110, wearable device 130, external display device 173, and mouse 174) via one or more network interfaces 404 (wired or wireless) and one or more networks 150, such as the Internet, other wide area networks, local area networks, metropolitan area networks, and so on;
- a web browser 426 (or other application capable of displaying web pages), which enables a user to communicate over a network with remote computers or devices;
- a wearable interface module 428, for interfacing with various modules and components of the wearable device 130. In some embodiments, the wearable interface module 428 includes:
   ∘ a driver 430, which provides two-way telemetry between the computing device and the wearable device 130 and data input for application 440;
- a cloud interface module 432, for providing two-way communication between the computing device 140 and the server system 160;
- an application 440. In some embodiments, the application 440 includes:
   ∘ a user interface 442 for providing visual information to users and for capturing user inputs;
   ∘ a signal input module 444 for receiving brain signals (e.g., electrical signals or data streams) of a human subject 102. As discussed with reference to Figures 1, 2, and 3, in some embodiments, the brain signals are collected by implant device 110 and transmitted to the wearable device 130. In some embodiments, the brain signals include attempted or intended speech signals (e.g., the human subject 102 tries to speak, thereby producing electrical signals corresponding to the intended movement of specific individual muscles or groups of muscles used for speech articulation, but their speech may not be vocalized due to underlying pathology preventing correct movement of the intended muscles or muscle groups). In some embodiments, the brain signals include attempted non-speech motor signals, such as attempted head, arm, hand, foot or leg movement signals (e.g., the human subject 102 tries to move their head or limbs, thereby producing electrical signals corresponding to the intended movement of specific individual muscles or groups of muscles used for the desired movement, but actual, physical movement may or may not occur due to underlying pathology preventing correct movement of the intended muscle or muscle groups) and attempted gestures (e.g. a hand squeeze or gesture);
   ∘ a signal processing module 446 for decoding the brain signals, including extracting electrophysiological features from the brain signals and performing signal processing functions. The extracted features represent brain activities intended for desired actions. In some embodiments, the signal processing module 446 analyzes signal characteristics such as amplitude, latency of event-evoked potentials, frequency power spectra (e.g., sensorimotor rhythms), or neuronal firing rates, in time-domain and/or frequency-domain. In some embodiments, the signal processing module 446 applies mathematical operations to enhance, filter, or modify the signals, allowing for better interpretation and utilization of the data;
   ∘ an interpretation module 448 (e.g., decoding module) for decoding the brain signals by recognizing patterns of the extracted features and the intended actions they represent. In some embodiments, the interpretation module 448 implements a classification process to recognize patterns in the extracted features. In some embodiments, the interpretation module 448 applies one or more machine learning models (e.g., data processing models 480 and/or data processing models 568) to decode the brain signals and determine intended actions of the human subject 102. In some embodiments, the interpretation module 448 applies a machine learning algorithm to decode the brain signals and determine intended actions of the human subject 102. In some embodiments, the machine learning algorithm is associated with the one or more machine learning models. In some embodiments, the interpretation module 448 is configured to generate a respective distinct set of intended actions depending on the operating mode (e.g., operating modes 190) of the computing device. In some embodiments, the interpretation module 448 is configured to translate and/or transform the classified features into actual commands;
   ∘ an output module 450 for controlling one or more devices, applications, and/or application interfaces to execute the one or more intended actions. In some embodiments, the output module 450 is configured to control the one or more devices, applications, and/or application interfaces to perform dictation control 452, keyboard control 454, cursor control 456, speech control 458, command control 460, accessibility control 462, and alert control 464. In some embodiments, the output module 450 is configured to control an external robotic arm. In some embodiments, the output module 450 is configured to control a prosthetic limb of the human subject 102; and
   ∘ a display generation module 466 for generating visual outputs for display on a display device such as a display screen 170 of the computing device 140 of an external display device 173;
- one or more other applications 468 that are executed on the computing device 140, Exemplary applications can include a messaging application, an email application, a data presentation and communication application;
- a training module 472 for performing limited machine learning model training. In some embodiments, the server system 160 is configured to train one or more machine learning models (e.g., decoding models) that are deployed locally on the computing device 140. In some embodiments, limited model training occurs locally on the computing device 140. In some embodiments, the training module 472 includes:
   ∘ one or more training datasets 474; and
   ∘ a training task repository 476, which includes training activities or tasks for training the models one or more data processing models 480.
- a data store 478 for storing data associated with the computing device 140, including:
   ∘ one or more data processing models 480 (e.g., machine learning models, machine learning algorithms, decoding models, and/or decoding algorithms) for supporting the functions performed by the signal processing module 446 and/or the interpretation module 448. As used herein, a data processing model 480 can refer a machine learning model or an algorithm; and
- APIs 482 for receiving API calls from one or more applications (e.g., a web browser 426, application 440, other applications 468), translating the API calls into appropriate actions, and performing one or more actions.

Each of the above identified executable modules, applications, or sets of procedures may be stored in one or more of the previously mentioned memory devices, and corresponds to a set of instructions for performing a function described above. The above identified modules or programs (i.e., sets of instructions) need not be implemented as separate software programs, procedures, or modules, and thus various subsets of these modules may be combined or otherwise re-arranged in various embodiments. In some embodiments, the memory 406 stores a subset of the modules and data structures identified above. Furthermore, the memory 406 may store additional modules or data structures not described above. In some embodiments, a subset of the programs, modules, and/or data stored in the memory 406 is stored on and/or executed by a server system 160.

Although Figure 4 shows a computing device 140, Figure 4 is intended more as a functional description of the various features that may be present rather than as a structural schematic of the embodiments described herein. In practice, and as recognized by those of ordinary skill in the art, items shown separately could be combined and some items could be separated. In addition, some of the programs, functions, procedures, or data shown above with respect to the computing device 140 may be stored or executed on a server system 160.

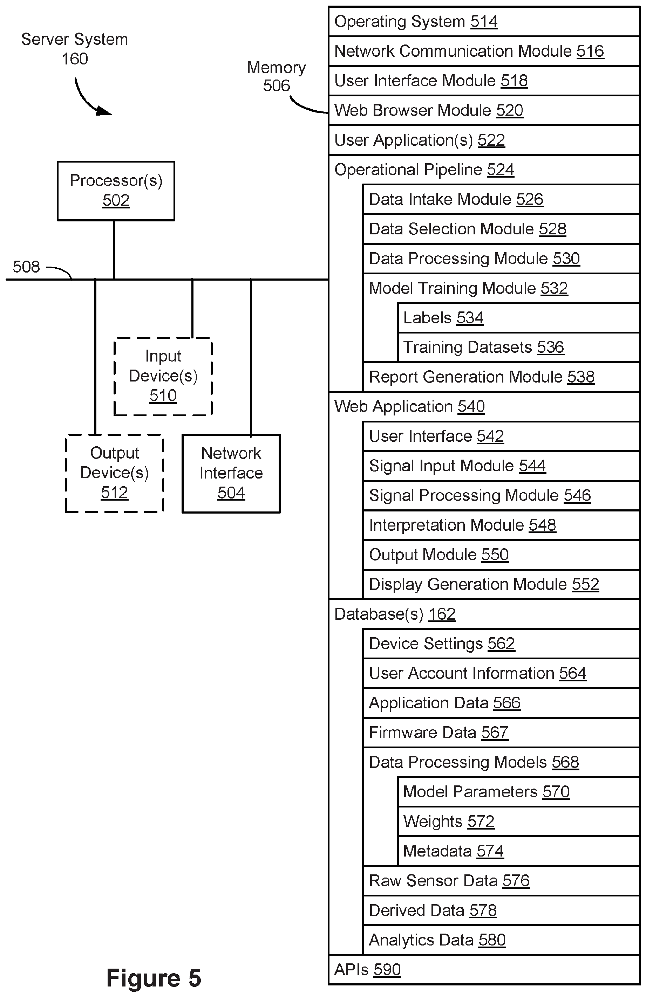

Figure 5 is a block diagram of a server system 160, in accordance with some embodiments. The server system 160 typically includes one or more processors 502 (e.g., processing units or cores (CPUs)), one or more network interfaces 504, memory 506, and one or more communication buses 508 for interconnecting these components (sometimes called a chipset). In some embodiments, the server system 160 includes one or more input devices 510, which facilitate user input, such as a keyboard, a mouse, a voice-command input unit or microphone, a touch screen display, a touch-sensitive input pad, a gesture capturing camera, or other input buttons or controls. In some embodiments, the server system 160 uses a microphone and voice recognition or a camera and gesture recognition to supplement or replace the keyboard. In some implementations, the server system 160 includes one or more cameras, scanners, or photo sensor units for capturing images. In some implementations, the server system 160 includes one or more output devices 512, which enable presentation of user interfaces and display content, including one or more speakers and/or one or more visual displays.

The memory 506 includes high-speed random access memory, such as DRAM, SRAM, DDR RAM, or other random access solid state memory devices. In some implementations, the memory 506 includes non-volatile memory, such as one or more magnetic disk storage devices, one or more optical disk storage devices, one or more flash memory devices, or one or more other non-volatile solid state storage devices. In some implementations, the memory 506 includes one or more storage devices remotely located from the processing units 502. The memory 506, or alternatively the non-volatile memory within the memory 506, includes a non-transitory computer readable storage medium. In some implementations, the memory 506, or the non-transitory computer readable storage medium of the memory 506, stores the following programs, modules, and data structures, or a subset or superset thereof:
- an operating system 514, which includes procedures for handling various basic system services and for performing hardware dependent tasks;
- a network communication module 516, which connects the server system 160 to other devices (e.g., various servers in the server system 160, computing device 140, a client device, or other devices) via one or more network interfaces 504 (wired or wireless) and one or more networks 150, such as the Internet, other wide area networks, local area networks, metropolitan area networks, and so on;
- a user interface module 518, which enables presentation of information (e.g., a graphical user interface for presenting applications, widgets, websites and web pages thereof, and/or games, audio and/or video content) at a computing device 140 or electronic device 178;
- a web browser module 520 for navigating, requesting (e.g., via HTTP), and displaying websites and web pages thereof, including a web interface for logging into a user account associated with a computing device 140 or another electronic device, controlling the computing device or electronic device if associated with the user account, and editing and reviewing settings and data that are associated with the user account;
- an operational pipeline 524, including:
   ∘ a data intake module 526 for obtaining respective neural signals that are collected by respective implant devices;
   ∘ a data selection module 528 for automatically choosing the most relevant and suitable data subsets from a larger data pool (e.g., form the one or more databases 162);
   ∘ a data processing module 538 for transforming raw sensor data 576 through various processing steps such as data cleaning, normalization, and feature extraction. In some embodiments, the data processing module 538 generates training data for use by a model training module 532 by preparing the processed data in a suitable format;
   ∘ the model training module 532 for receiving the training data and establishing one or more data processing models 568 for processing neural data collected by implant devices 110. In some embodiments, the model training module 532 includes labels 534 for training the data processing models 568. In some embodiments, the model training module 532 includes training datasets 536 for training the data processing models 568;
- a web application 540, which may be downloaded and executed by a web browser 426 on a user's computing device 140. In general, a web application 540 has the same functionality as application 440, but provides the flexibility of access from any device at any location with network connectivity, and does not require installation and maintenance. In some embodiments, the web application 540 includes various software modules to perform certain tasks, such as:
   ∘ a user interface 542, which provides the user interface for all aspects of the web application 540;
   ∘a signal input module 544, which has the same functionalities as signal input module 444
   ∘ a signal processing module 546, which has the same functionalities as signal processing module 446;
   ∘ an interpretation module 548, which has the same functionalities as interpretation module 448;
   ∘ an output module 550, which has the same functionalities as output module 450; and
   ∘ a display generation module 552, which has the same functionalities as display generation module 466;
- one or more databases 162 for storing at least data including one or more of:
   ∘ device settings 562 including common device settings (e.g., service tier, device model, storage capacity, processing capabilities, communication capabilities, etc.) of the one or more server systems 160, computing devices 140, or other devices;
   ∘ user account information 564 for the application 440 and/or the web application 540, such as user names, security questions, account history data, user preferences, and predefined account settings
   ∘ application data 566 from the application 540, web application 540, and/or the user applications
   ∘ firmware data 567 corresponding to respective firmware versions;
   ∘ data processing models 568 (e.g., machine learning models, machine learning algorithms, decoding models, and/or decoding algorithms) for decoding neural signal data into intended user actions. In some embodiments, the data processing models 568 include model parameters 570, weights 572, and metadata 574
   ∘ raw sensor data 576 acquired by one or more electrode arrays 208;
   ∘ derived data 578 obtained by transforming, processing, or analyzing the raw sensor data 576; and
   ∘ analytics data 580, which are data that are collected, processed, and analyzed from the raw sensor data 576 and the derived data 578 to gain insights and identify patterns; and
- APIs 590 for receiving API calls from one or more applications (e.g., web browser module 520, user applications 522, and web application 540) and the one or more data processing models 568, translating the API calls into appropriate actions, and performing one or more actions.

Each of the above identified executable modules, applications, or sets of procedures may be stored in one or more of the previously mentioned memory devices, and corresponds to a set of instructions for performing a function described above. The above identified modules or programs (i.e., sets of instructions) need not be implemented as separate software programs, procedures, or modules, and thus various subsets of these modules may be combined or otherwise re-arranged in various embodiments. In some embodiments, the memory 506 stores a subset of the modules and data structures identified above. Furthermore, the memory 506 may store additional modules or data structures not described above.

Although Figure 5 shows a server system 160, Figure 5 is intended more as a functional description of the various features that may be present rather than as a structural schematic of the embodiments described herein. In practice, and as recognized by those of ordinary skill in the art, items shown separately could be combined and some items could be separated. In addition, some of the programs, functions, procedures, or data shown above with respect to a server system 160 may be stored or executed on a computing device 140. In some embodiments, the functionality and/or data may be allocated between a computing device 140 and one or more server systems 160. Furthermore, one of skill in the art recognizes that Figure 5 need not represent a single physical device. In some embodiments, the server functionality is allocated across multiple physical devices in a server system. As used herein, references to a "server" include various groups, collections, or arrays of servers that provide the described functionality, and the physical servers need not be physically colocated (e.g., the individual physical devices could be spread throughout the United States or throughout the world).

### Methods for Operating a Brain Computer Interface (BCI) System in Safe Mode

As described above with reference to Figures 1 to 5, a BCI system can apply machine learning algorithms to decode brain signals of a human subject to determine one or more intended actions of the human subject. In some circumstances, the algorithms employed in a BCI system may produce erroneous or inaccurate predictions of user intent. For example, poor BCI performance may occur in the first instance where the algorithms have not been fully calibrated, or when recalibration is necessary because algorithm performance has degraded due to a number of circumstances such as electrical noise or artifacts, degradation of electrodes, or the development of scar tissue around the electrodes, neuroplasticity, latency, and errors in software or communications.

As disclosed, in some situations where the algorithms employed in a BCI system produces erroneous or inaccurate predictions of user intent, the computing device may switch a mode of operation from a normal mode of operation (e.g., high performance mode 191) to a safe mode of operation (e.g., safe mode 193). In some embodiments, in the safe mode of operation, partial control of the computing device may be possible through a simplified user interface (e.g., safe mode user interface 710). "Safe mode" is crucial for BCI systems because it allows the system to transition to a predetermined state of lesser (e.g., reduced or limited) functionality and/or sensitivity to brain signals in case of a malfunction or error, thereby minimizing potential harm or frustration to the patient by ensuring that the device does not deliver unexpected or inappropriate results when issues arise. Essentially, the safe mode of operation prioritizes patient safety and performance of high-priority actions, by providing a fallback mechanism when normal operation is compromised.

As disclosed, in some embodiments, safe mode operation involves rudimentary cursor control when more fine-grained controls or speech are not available or not yet trained. When full control of a computer is not possible due to the inadequacy of the current signal training, the safety mode may be able to provide partial usability. The main benefits here are that a user can still retain partial control of the computing device when full control is not feasible. As disclosed, in some embodiments, the user can provide reinforcement learning input to the computing device, to work toward bootstrapping the full system back up.

In some embodiments, while operating in safe mode, functionality of the BCI system is limited. For example, in some embodiments, while operating in safe mode, the BCI system is configured to turn off speech decoding functionality. In some embodiments, while operating in safe mode, cursor control prediction limited to movement in only a subset of directions (e.g., up, down, left, and right directions; or up and down directions). In some embodiments, while operating in safe mode, the default position of the cursor is at the center of the user interface. In some embodiments, while operating in safe mode, classification dimensionality is reduced.

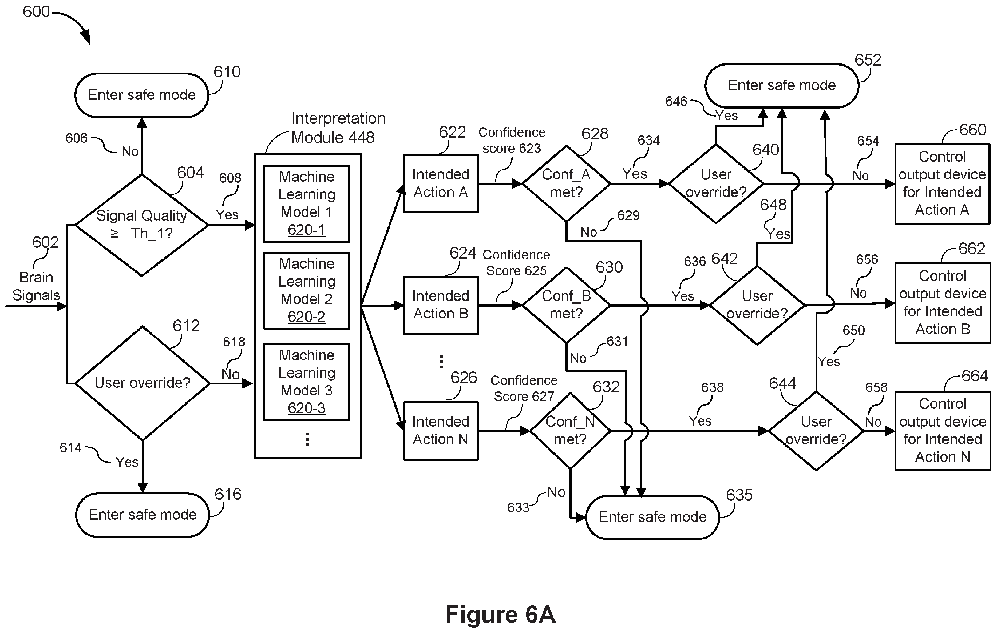

Figure 6A illustrates a workflow 600 performed by computing device 140 (e.g., via processor(s) 402) when the computing device 140 is operating in a normal mode of operation, in accordance with some embodiments. In accordance with some embodiments, the normal mode of operation is also referred to as a high-performance mode (e.g., high performance mode 191).

In the workflow 600, the computing device 140 receives brain signals (602) of a human subject (e.g., human subject 102). As discussed in Figure 1A, in some embodiments the computing device 140 can receive brain signals (e.g., via signal input module 444) from implant device 110 that is implanted into the skull of the human subject and/or a wearable device 130 that is communicatively connected to the implant device 110. In some embodiments, the brain signals 602 include a signal associated with an attempt by the human subject to speak. In some embodiments, the brain signals 602 includes a signal associated with an attempt by the human subject to move a body part of the human subject. Example body parts can include and are not limited to a digit (e.g., a finger or a toe), a limb, one or more facial features (e.g., eyes, nose, mouth), the head, or a portion of a torso of the human subject. In some embodiments, the brain signals 602 includes a signal associated with an attempt by the human subject to control a facial expression of the human subject. In some embodiments, the brain signals 602 includes a signal associated with an attempt by the human subject to control an external device. Example external devices include and are not limited to one or more smart home devices located in the same premises as the human subject, such as a smart thermostat or a smart camera, or a robotics arm, a prosthetic limb of the human subject, the computing device (e.g., one or more applications executing on the computing device), and/or peripheral devices associated with the computing device such as keyboard 176, mouse 174, or touchpad 175)

With continued reference to Figure 6A, the computing device 140 determines, in step 604 (e.g., via signal processing module 446), whether a signal quality of the received brain signals satisfies a threshold quality score Th_1 (e.g., a value or a number). For example, the computing device 140 can determine a quality score for the received signals by applying a quality metric such a signal-to-noise ratio (SNR), data packet loss, latency (e.g., time it takes for signal to travel from implant device 110 to wearable device 130, or from wearable device 130 to the computing device 140), jitter, interference with other devices, and/or spurious emissions. In accordance with some embodiments, when the signal quality of the received brain signals does not satisfy the threshold quality score Th_1 (step 606) (e.g., the signal quality is less than or equal to a threshold quality score), the computing device 140 transitions (e.g., switches) from the normal mode of operation to a safe mode of operation (step 610: enter safe mode) (e.g., safe mode 193). In some embodiments, the computing device 140 can also enter the safe mode of operation (step 616, via step 614) in accordance with receiving a user override instruction (612). For example, the user override instruction 612 can be an instruction to the computing device 140 (via step 614) from the human subject 102, or a caregiver of the human subject, or a medical practitioner, to operate the computing device in the safe mode. In some embodiments, the threshold quality score (Th_1) is a score (e.g., a value) that is averaged over time. In some embodiments, the threshold quality score that is computed according to other time-based heuristics. For example, a one-time low performance may not trigger safety mode, but similar low performance persisting for a few minutes may trigger safety mode.

In some embodiments, in accordance with a determination that signal quality of the received brain signals satisfies a threshold quality score Th_1 (step 608) (e.g., the signal quality is greater than or equal to a threshold quality score) and/or no user override instruction 612 has been received, the computing device 140 decodes the brain signals 602 via interpretation module 448. In some embodiments, the computing device 140 (e.g., the interpretation module 448) executes a machine learning algorithm that is configured to process the brain signals 602 to obtain one or more intended actions (e.g., intended action A 622, intended action B 624, and intended action N 626) of the human subject 102. In some embodiments, the machine learning algorithm is associated with one or more machine learning models, such as machine learning models 620-1, 620-2, and 620-3 as depicted in Figure 6A. In some embodiments, a respective learning model is a decoder model (e.g., a deep learning model) that is configured to output representations (e.g., vector representations) corresponding to a respective output control modality. In some embodiments, the machine learning models 620 are trained according to data from the human subject 102. In some embodiments, the machine learning models 620 are specifically trained using data from the human subject 102. This way, the machine learning model can determine intended actions tailored to the unique preferences, behaviors, and past interactions the human subject 102, effectively creating a personalized experience based on the specific data profile of the human subject 102. In some embodiments, the one or more machine learning models 620 comprise multiple decoder models, each of which is configured to output representations corresponding to one specific output control modality. In some embodiments, the one or more machine learning models 620 comprise a single decoder model that is configured to output representations corresponding to multiple output control modalities.

In accordance with some embodiments, for a respective intended action, the computing device 140 determines a respective confidence score corresponding to the determined intended action and determines whether the respective confidence score satisfies a respective threshold confidence score (e.g., value or level). For example, the respective confidence score represents a confidence level (e.g., likelihood or probability) that the intended action of the human subject 102 has been correctly decoded by the computing device 140. In some embodiments, when the confidence score for a respective decoded intended action satisfies the respective threshold confidence score, and no user override instruction has been received, the computing device 140 controls a respective output device corresponding to the respective decoded intended action. In some embodiments, when the confidence score for a respective decoded intended action does not satisfy the respective threshold confidence score, the computing device 140 transitions from the normal mode of operation to the safe mode of operation.

Referring to decoded intended action A 622 in the workflow 600, in some embodiments the computing device 140 determines a confidence score 623 representing a confidence (e.g., likelihood or probability) that the intended action of the human subject, based on the received brain signals, is the decoded intended action A. The computing device 140 determines whether the confidence score 623 satisfies a respective threshold value (Conf_A) in step 628. In some embodiments, in accordance with a determination that the confidence score 623 does not meet the respective threshold value (step 629) (e.g., the confidence score 623 is less than or equal to Conf_A), the computing device 140 enters the safe mode of operation (as shown in step 635. In some embodiments, in accordance with a determination that the confidence score 623 satisfies the respective threshold value (step 634) (e.g., the confidence score 623 is greater than or equal to Conf_A), but a user override instruction 640 has been received in step 646, the computing device 140 enters the safe mode of operation as shown in step 652. In some embodiments, in accordance with a determination that the confidence score 623 satisfies the respective threshold value (step 634) (e.g., the confidence score 623 is greater than or equal to Conf_A), and that no user override instruction 640 has been received (step 654), the computing device 140 proceeds to step 660, where the computing device 140 controls an output device for intended action A.

A similar situation applies for decoded intended action B 624 and decoded intended action N 626 in the workflow 600. In some embodiments, for decoded intended action B 624, the computing device 140 determines a confidence score 625 representing a confidence that decoded intended action B is the intended action of the human subject 102. The computing device 140 determines whether the confidence score 625 satisfies a respective threshold value (Conf_B) in step 630. In some embodiments, in accordance with a determination that the confidence score 625 does not meet the respective threshold value (e.g., the confidence score 625 is less than or equal to Conf_B) in step 631, the computing device 140 enters the safe mode of operation as shown in step 635. In some embodiments, in accordance with a determination that the confidence score 625 satisfies the respective threshold value (e.g., the confidence score 625 is greater than or equal to Conf_B) in step 636, but a user override instruction 642 has been received in step 648, the computing device 140 enters the safe mode of operation as shown in step 652. In some embodiments, in accordance with a determination that the confidence score 625 satisfies the respective threshold value (e.g., the confidence score 625 is less than or equal to Conf_B) in step 636, and that no user override instruction 640 has been received by the computing device 140 (step 656), the computing device 140 proceeds to step 662, where the computing device 140 controls an output device for intended action B.

In some embodiments, for decoded intended action N 626, the computing device 140 determines a confidence score 627 representing a likelihood that decoded intended action N is the intended action of the human subject 102. The computing device 140 determines whether the confidence score 627 satisfies a respective threshold value (Conf_N) in step 632. In some embodiments, in accordance with a determination that the confidence score 627 does not meet the respective threshold value (e.g., the confidence score 627 is less than or equal to Conf_N) in step 633, the computing device 140 enters the safe mode of operation as shown in step 635. In some embodiments, in accordance with a determination that the confidence score 627 satisfies the respective threshold value in step 638 (e.g., the confidence score 627 is greater than or equal to Conf_N), but a user override instruction 644 has been received in step 650, the computing device 140 enters the safe mode of operation as shown in step 652. In some embodiments, in accordance with a determination that the confidence score 627 satisfies the respective threshold value in step 638 (e.g., the confidence score 627 is greater than or equal to Conf_N), and that no user override instruction 640 has been received by the computing device 140 (step 658), the computing device 140 proceeds to step 664, where the computing device 140 controls an output device for intended action N.

In some embodiments, the output device for an intended action can be the computing device 140 itself. For example, in some embodiments, the computing device 140 can control itself by generating (e.g., via display generation module 466) a user interface (e.g., user interface 442) and displaying the user interface 442 on a display device associated with the computing device 140. In some embodiments, the computing device 140 can control itself by activating one or more third-party applications (e.g., other application(s) 468) that are installed on the computing device 140.

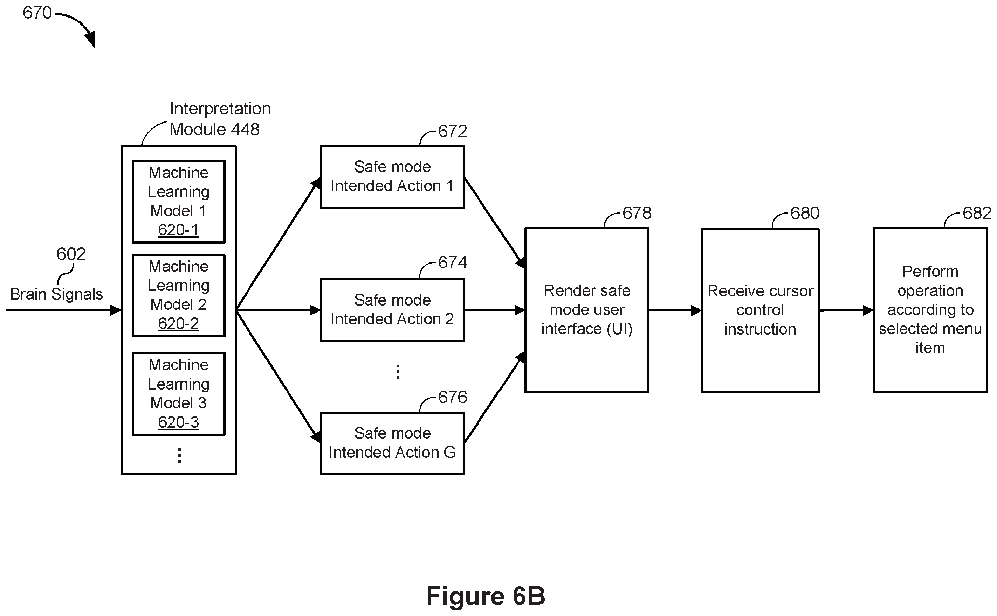

Figure 6B illustrates a workflow 670 performed by computing device 140 (e.g., via processor(s) 402) when the computing device 140 is operating in a safe mode of operation (e.g., safe mode 193), in accordance with some embodiments. As disclosed, the safe mode limits operations of the computing device 140 to a subset of operations, less than all of a plurality of operations that the computing device is configured to perform in the normal mode.

Referring to Figure 6B, in some embodiments, while operating in safe mode, the computing device 140 receives brain signals 602 and inputs the received signals into interpretation module 448, which executes a machine learning algorithm that is configured to process the brain signals 602 to obtain one or more intended actions of the human subject 102. For example, as described with reference to Figures 4 and 6A, the interpretation module 448 can apply one or more machine learning models such as machine learning models 620-1, 620-2, and 620-3 to decode the brain signals. These details are not repeated here for the sake of brevity.

With continued reference to Figure 6B, while operating in the safe mode, the computing device 140 (e.g., interpretation module 448) is configured to output a set of (e.g., one or more) safe mode intended actions (e.g., safe mode intended actions 672, 674, and 676) of the human subject based on decoding the signals. For example, Figure 6B shows the interpretation module 448 output a first safe mode intended action 672, a second safe mode intended action 674, and a G^{th} safe mode intended action 676. In some embodiments, the set of safe mode intended actions (e.g., safe mode intended actions 672, 674, and 676) is a subset of the intended actions (e.g., intended actions 622, 624, and 626) that are output by the computing device 140 when the computing device operates in the normal mode. In some embodiments, the set of safe mode intended actions are different from the intended actions that are output by the computing device 140 when the computing device is operating in the normal mode of operation. In some embodiments, the set of safe mode intended actions correspond to signals that the computing device 140 can decode with the highest level of confidence.

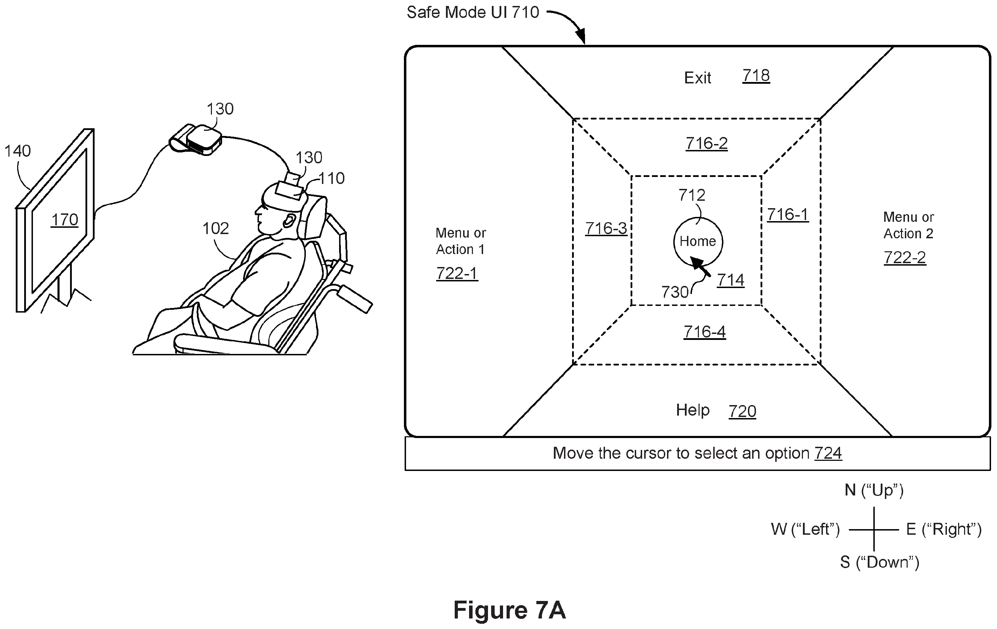

In some embodiments, while operating in the safe mode, partial control of the computing device 140 may be possible through a simplified safe mode user interface. Figure 6B shows that in step 678, the computing device 140 renders (e.g., generates and displays) a safe mode user interface (UI) (e.g., safe mode UI 710, Figures 7A to 7F) based on the set of safe mode intended actions, and displays the safe mode user interface on a display of the computing device 140. In some embodiments, the safe mode user interface includes a set of one or more affordances (e.g., user-selectable input controls) corresponding to the subset of operations that the computing device 140 is configured to perform in the safe mode. In step 680, the computing device 140 receives a cursor control instruction to control a cursor in the safe mode user interface. In some embodiments, the computing device 140 performs an operation according to a selected menu item in the safe mode user interface as shown in step 682.

Figures 7A to 7F illustrate a safe mode user interface (UI) 710, in accordance with some embodiments. The safe mode UI 710 is a user interface that is displayed on a display 170 of the computing device 140 when the computing device 140 is operating in safe mode. In some embodiments, the safe mode UI 710 facilitates rudimentary cursor control. For example, Figures 7A to 7F depict that the safe mode UI 710 is divided into four "quadrants" and each quadrant includes respective input controls (e.g., options or affordances).

In some embodiments, the safe mode UI 710 includes a Home region 712, corresponding to a starting portion of a cursor 730. The Home region 712 is located approximately at the center of the safe mode UI 710. A user (e.g., human subject 102) can send a brain signal to the computing device 140 (via implant device 110 → wearable device 130 → computing device 140) to instruct the computing device 140 to move the cursor 730 in one of "up," "down," "left," or "right" directions, to a menu or option corresponding to the user's intended input control. It should be noted that the cursor movement is controlled/performed/executed by the computing device 140 based on intended actions of the human subject 102, which are determined according to decoded brain signals of the human subject 102. The human subject 102 does not interact with any pointer device to effect cursor movement.

In some embodiments, and as described in Figure 6B, the computing device 140 decodes the brain signals of the human subject 102 into a set of (e.g., one or more) safe mode intended actions when it is operating in safe mode. The computing device 140 displays the set of safe mode intended actions as menu (or action) input controls as regions 722-1 and 722-2 of the safe mode UI 710, as illustrated in Figure 7A. Figure 7A also shows that in some embodiments, the safe mode UI 710 includes a region 718 that, when "selected" (e.g., when the cursor 730 moves to that region), causes the computing device 140 to exit safe mode. In some embodiments, the safe mode UI 710 includes a region 720 that, when "selected" (e.g., when the cursor 730 moves to that region), causes the computing device 140 to trigger a call for help, for example to a caregiver of the human subject 102, or to a medical practitioner, or to place a 9-1-1 call.

In some embodiments, the safe mode UI 710 includes a reinforcement learning region 714 that is immediately adjacent to and surrounds the Home region 712. The reinforcement learning region 714 is used by the training module 472 of the computing device 140 for reinforcement learning. The bigger picture around reinforcement learning is that the user performs some action, receives visual feedback, and then makes a choice as to whether to continue that action or not. The user's choice to continue in that action results in positive reinforcement for the training module 472 and the user's choice not to continue that action results in negative reinforcement for the training module

As a first example of reinforcement learning, suppose that the human subject 102 attempts to move the cursor "up" or northward and the interpretation module 448 outputs an intended action to control movement the cursor 730 in the "left" direction based on decoding the brain signals of the human subject. In response to receiving this output from the interpretation module, the computing device 140 controls movement of the cursor 730 from the Home region 712 to the reinforcement learning region 714 in a "left" direction (e.g., westward direction). Suppose also that as the computing device 140 is controlling (or has controlled) movement of the cursor 730 from the Home region 712 to the reinforcement learning region 714 in the "left" direction when it receives a subsequent instruction from the human subject 102 (by decoding the brain signals from the human subject) to control movement of the cursor in another direction (e.g., to move to the "Up" or northward direction) or to stop moving the cursor 730. The termination of the attempt to move the cursor leftwards corresponds to negative reinforcement because it indicates to the computing device 140 that the computing device 140 may have decoded the brain signals incorrectly.

As a second example of reinforcement learning, suppose that the human subject 102 attempts to move the cursor "down" and the interpretation module 448 outputs an intended action to control movement the cursor 730 in the "down" (e.g., southward) direction based on decoding the brain signals of the human subject. In response to receiving this output from the interpretation module, the computing device 140 controls movement of the cursor 730 from the Home region 712 to the reinforcement learning region 714 in a "down" direction (e.g., southward direction). Suppose also that as the computing device 140 is controlling (or has controlled) movement of the cursor 730 from the Home region 712 to the reinforcement learning region 714 in the downward direction when it receives a signal from the human subject 102 to continue movement of the cursor into the region 716-4 (i.e., the same downward direction). This indicates positive reinforcement because it indicates to the computing device 140 that the interpretation module 448 has decoded the brain signals correctly. In this second example, the computing device 140 can infer positive feedback for the intended cursor direction "down."

In some embodiments, there need not be a "perfect alignment" between cursor movement and intended action, but so long as there is some correction between cursor movement direction and labels for intended actions, reinforcement learning is possible. In some embodiments, as a user (e.g., human subject 102) interacts with the safe mode UI 710 by sending brain signals to the computing device 140 to control movement of the cursor 730, reinforcement learning happens in the background processes of the computing device 140 and cursor control naturally improves without the need for an explicit training session.

In some embodiments, the safe mode UI 710 includes a context region 716, depicted as four quadrants 716-1 to 716-4 in Figure 7A. For example, when the cursor 730 position is moved by the computing device 140 (via user instruction) into the context region 716, the safe mode UI 710 can display additional information about the related action (e.g. which submenus are available or details about what result would be triggered) when cursor movement continues in the same direction. As an example, when the cursor 730 is positioned in the context region 716-1, the safe mode UI 710 can display a tooltip indicating additional information about menu or action 722-2.

In some embodiments, by causing the computing device 140 to control movement of the cursor 730 through the context region into one of the regions 718, 720, 722-1, and 722-2, a user selects that direction using "depth" rather than click or dwell time (e.g., moving the cursor 730 into one of the regions 718, 720, 722-1, and 722-2 indicates selection of the option).

In some embodiments, the safe mode UI 710 displays a status bar 724 that provides information to the human subject 102.

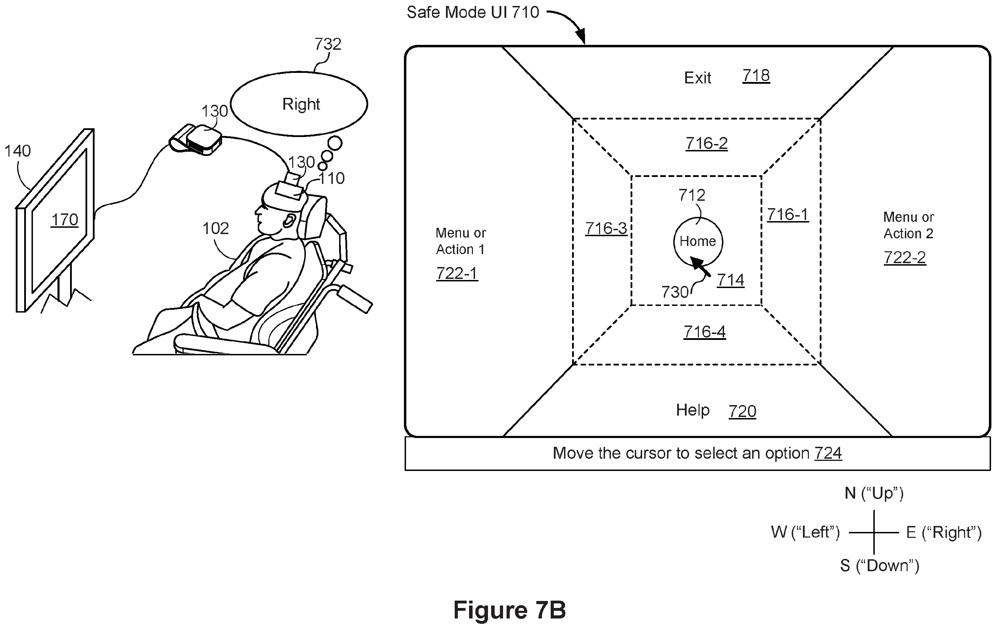

Figure 7B illustrates an example where the computing device 140 decodes brain signals 732 received from the human subject 102 and determines, based on the decoding, that the brain signals 732 include an instruction (e.g., intended action) by the human subject 102 to move the cursor to the right. In some embodiments, the computing device 140 includes one or more input devices 412, such as a camera, directed to the human subject 102, and the computing device performs the decoding based on a combination of received brain signals and other physical signals such as blinking of the eye of the human subject 102, or eye movement (e.g., in certain directions) of the human subject 102.

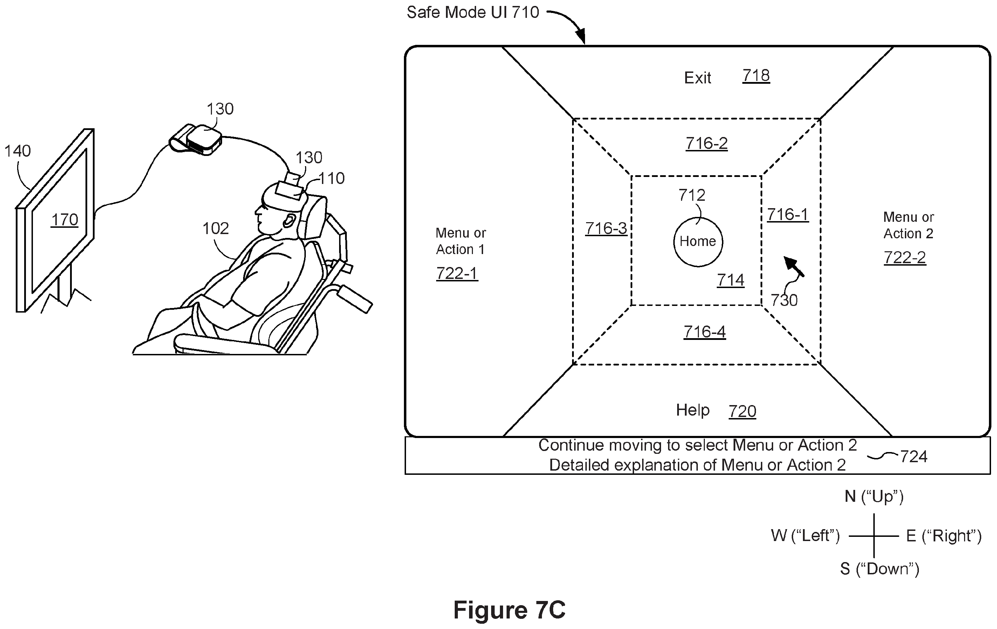

Figure 7C illustrates that in response to determining the instruction, the computing device 140 controls movement of the cursor from the Home Region 712, past the reinforcement learning region 714, into the context region 716-1. When the cursor 730 is in the context region 716-1, the safe mode UI 710 displays, in the status bar 724, an explanation indicating that continuing movement of the cursor into the region 722-2 will select menu or action 2. In some embodiments, the status bar 724 displays a detailed explanation of menu or action 2.

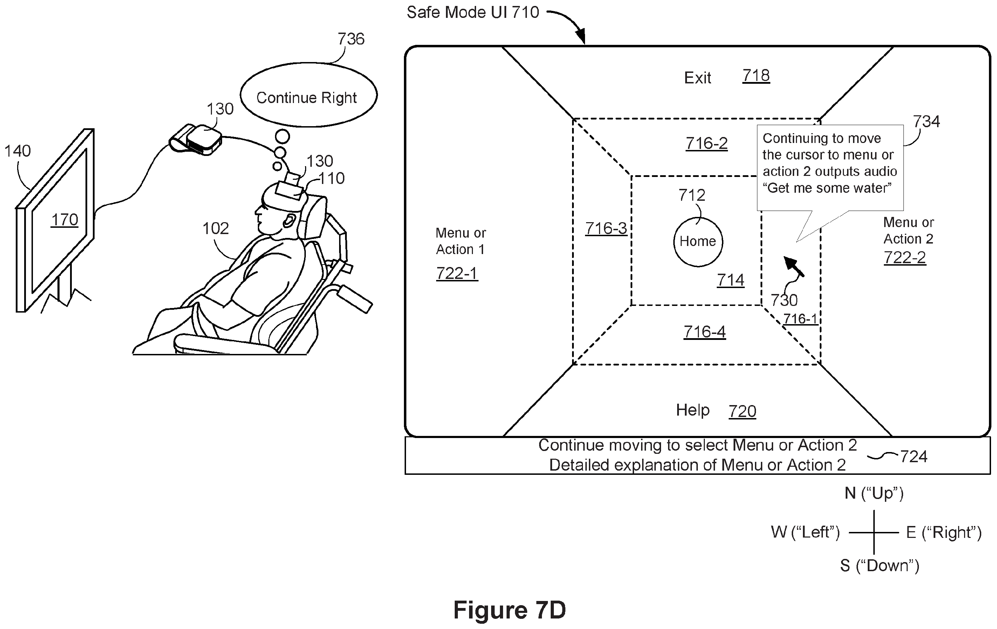

In some embodiments, and as illustrated in Figure 7D, the computing device 140 displays a tooltip 734 that provides a detailed explanation of what happens when the cursor moves into the region 722-2 corresponding to menu or option 2. In Figure 7D, the computing device 140 decodes another brain signal 736 from the human subject 102 and determines, based on the decoding, that the brain signal 736 corresponds to an instruction (e.g., intended action) by the human subject 102 to continue to move the cursor 730 to the right.

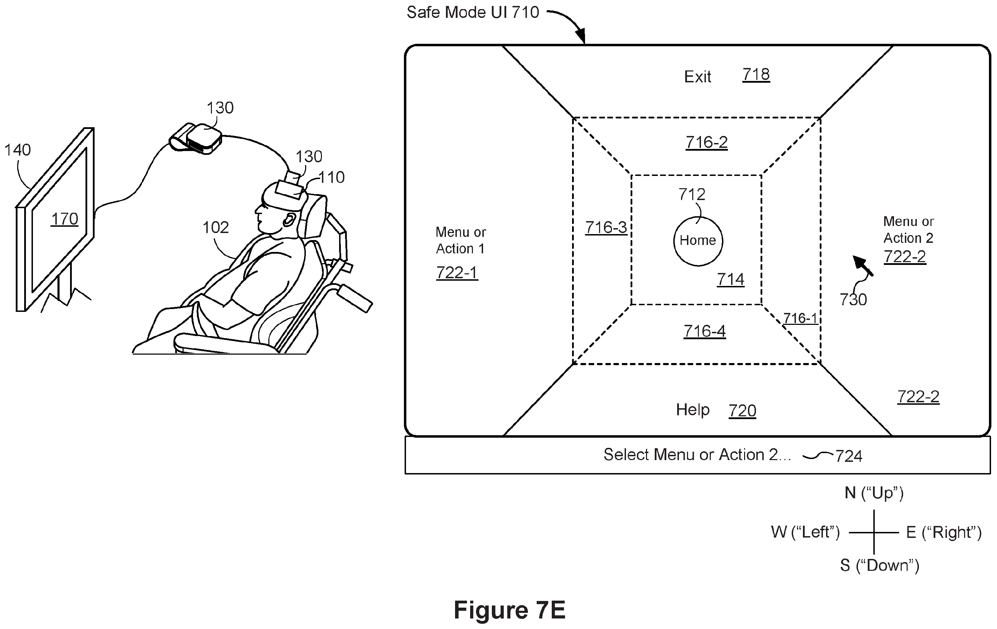

Figure 7E illustrates the computing device 140 moving the cursor 730. In some embodiments, moving the cursor 730 into the region 722-2 selects the menu or action that is displayed in the region 722-2. In some embodiments, the status bar 724 indicates selection of the menu or action, corresponding to the region 722-2.

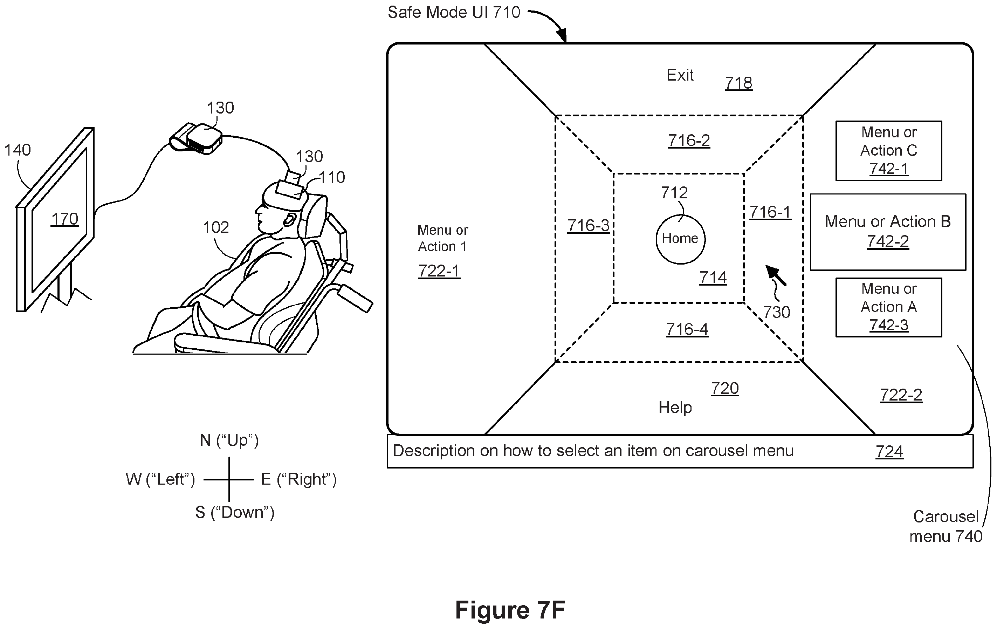

Figure 7F illustrates another example of the safe mode UI 710, in accordance with some embodiments. In this example, the region 722-2 displays multiple menu or action items 742 (e.g., menu or action items 742-1 to 742-3) as a carousel menu 740. Each menu item 742 is a user-selectable input control. For example, in some embodiments, the computing device 140 is configured to control the cursor 730 to move in left/right/up/down directions. Navigating left or right can correspond to an intended user action to rotate the carousel menu 740 (e.g., rotating menu or action items 742), whereas navigating down can correspond to an intended user action to select the item. In some embodiments, the computing device 140 is configured to control the cursor 730 to move just in the left or right directions. Navigating left can correspond to an intended user action to move to the next item on the carousel menu 740, whereas navigating right can correspond to an intended user action to select the item. In some embodiments, the computing device 140 is configured to control the cursor to move in one direction. The carousel menu 740 can be configured to rotate on a timer, and unary action corresponds to an intended action to select the item. In some embodiments, when the cursor 730 is in the context region 716-1, the safe mode UI 710 displays, in the status bar 724, an explanation on how to select an item on the carousel menu.

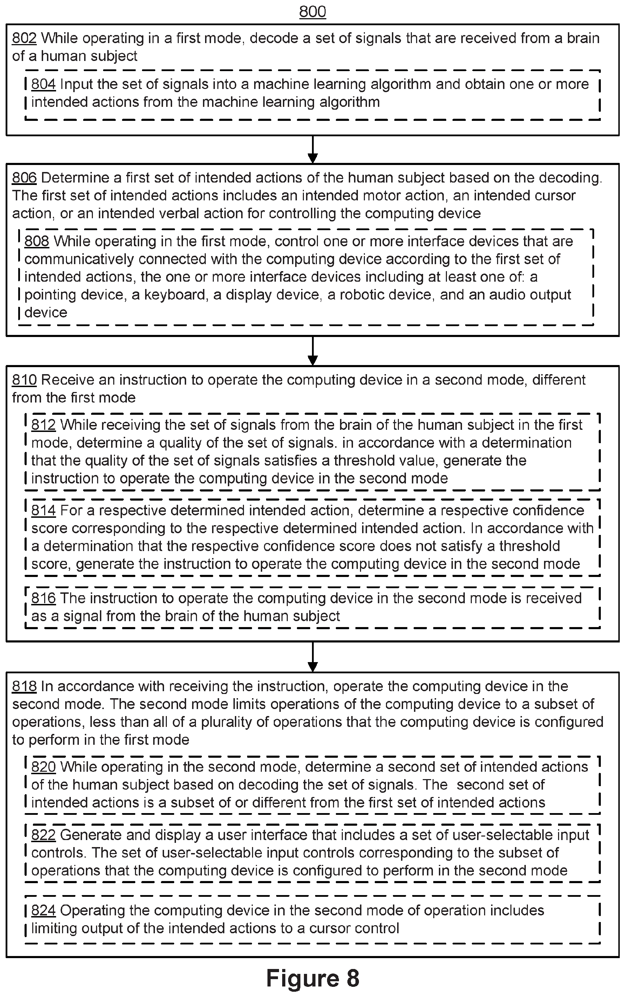

Figure 8 provides a flowchart of an example process for operating a computing device in safe mode, in accordance with some embodiments. The method 800 is performed at a computing device (e.g., computing device 140) that includes one or more processors (e.g., processor(s) 402) and memory (e.g., memory 406). The memory stores one or more programs configured for execution by the one or more processors. In some embodiments, the operations shown in Figures 1A to 1D, 2A to 2D, 3A, 3B, 6A, 6B, and 7A to 7F correspond to instructions stored in the memory 406 or other non-transitory computer-readable storage medium. The computer-readable storage medium may include a magnetic or optical disk storage device, solid state storage devices such as Flash memory, or other non-volatile memory device or devices. In some embodiments, the instructions stored on the computer-readable storage medium include one or more of: source code, assembly language code, object code, or other instruction format that is interpreted by one or more processors. Some operations in the method 800 may be combined with other operations, and/or the order of some operations may be changed.

As disclosed, operating a computing device in safe mode provides a technical improvement in the operation of a BCI system (and the associated computing device) under degraded neural-signal conditions. Rather than merely presenting information to a user, the computing device reconfigures how neural data are interpreted and how output commands are generated in response to a detected reduction in decoding reliability. For example, when the computing device detects that current decoding of the neural data no longer satisfies one or more reliability criteria, the computing device can automatically deactivate one or more higher-dimensional or lower-reliability decoding paths, deactivate one or more active applications or application features, and constrain output generation to a reduced set of machine-executable commands that are more robustly decodable from the available neural data. As a result, the computing device reduces unintended machine actions, reduces propagation of decoding errors to external applications or devices, and preserves a reliable communication pathway between the human subject and the computing device while the normal operating mode is unavailable.

Referring to Figure 8, the computing device, while operating in a first mode (e.g., normal mode or high performance mode 191), decodes (802) a set of signals that are received from a brain of a human subject (e.g., human subject 102). In some embodiments, the first mode corresponds to a high-performance operating mode (e.g., high performance mode 191) of the computing device. In some embodiments, the high performance mode 191 is also referred to as the normal mode of operation (or normal operating mode). In some embodiments, prior to the decoding, the computing device receives brain signals from the brain of the human subject via an implant device 110 and a wearable device 130, as illustrated in Figures 1A and 6A.

In some embodiments, the set of signals comprises a signal associated with an attempt by the human subject to speak. In some embodiments, the set of signals comprises a signal associated with an attempt by the human subject to move a body part of the human subject. In some embodiments, a signal associated with an attempt by the human subject to move a body part of the human subject is also referred to as an attempted non-speech signal or an attempted motor gesture. For example, the human subject can attempt to move a digit (e.g., finger or toe) of the human subject, or a limb of the human subject, or one or more facial features (e.g., eyes, nose, mouth) of the human subject, or the head of the human subject, or a portion of a torso of the human subject. In some embodiments, the set of signals comprises a signal associated with an attempt by the human subject to control a facial expression of the human subject (e.g., an attempted non-speech signal or an attempted motor gesture). In some embodiments, the set of signals comprises a signal associated with an attempt by the human subject to control an external device. Example external devices include, and are not limited to, one or more smart home devices such as a smart thermostat or a smart camera, or a robotic arm, or a computer device (e.g., computing device 140) and associated peripherals such as a keyboard 176 or a pointing device such as a mouse 174 or a touchpad 175.

With continued reference to Figure 8, in some embodiments, while operating in the first mode, the computing device decodes the set of signals (e.g., via interpretation module 448) that are received from the brain of a human subject by inputting (804) the set of signals into a machine learning algorithm and obtaining one or more intended actions from the machine learning algorithm. In some embodiments, the machine learning algorithm is associated with one or more machine learning models (e.g., machine learning models 620, data processing models 480, and/or data processing models 568). In some embodiments, the machine learning algorithm is associated with one or more machine learning models that are trained according to data from the human subject. In some embodiments, the one or more machine learning models are customized for the human subject. For example, in some embodiments, the one or more machine learning models are specifically trained using data from the human subject. Accordingly, the machine learning models can determine intended actions tailored to the unique preferences, behaviors, and past interactions the human subject, effectively creating a personalized experience based on their specific data profile.

In some embodiments, the machine learning algorithm comprises a plurality of decoder models (e.g., deep learning models) that are configured to output representations (e.g., vector representations) corresponding to a plurality of output control modalities. The plurality of output control modalities corresponds to a plurality of intended action categories. For example, the output control modalities can include a text modality, a speech modality, an audio (e.g., synthesized voice) modality, a visual modality, an effector control (e.g., controlling an end effector of a robotic arm), a selection control, and/or an alert control modality. In some embodiments, the plurality of output control modalities corresponds to a plurality of categories, such as an attempted speech category, a brain-to-text category, a cursor control category. In some embodiments, when the category is an intended speech category, the one or more machine learning models can decode the intended speech against a dictionary of 2,000 or more words. In some embodiments, there is a one-to-one correspondence between decoder model and modality, where one decoder model is configured to output representations for a respective corresponding modality. In some embodiments, there is a one-to-many correspondence between decoder model and modality, such that a first decoder model can be configured to output representations corresponding to at least two distinct modalities. In some embodiments, the machine learning algorithm comprises a single decoder model that is configured to output representations corresponding to multiple output control modalities.

In some embodiments, the set of signals includes an attempted speech signal (e.g., a signal associated with an attempt by the human subject to speak). In some embodiments, while operating in the first mode, the machine learning algorithm is configured to decode the attempted speech signal by identifying one or more words from a predefined corpus of words and construct one or more sentences from the one or more words. For example, the predefined corpus of words can include a collection of at least 2000 words, 5000 words, 10,000 words or 20,000 words.

In some embodiments, while operating in the first mode, all of the decoding are performed locally on the computing device, in real time, as the signals are received. In some embodiments, some of the decoding is performed locally on the computing device in real time whereas a subset of the decoding is performed by a server system (e.g., server system 160).

With continued reference to Figure 8, the computing device determines (806) (e.g., predicts) a first set of (e.g., one or more) intended actions of the human subject based on the decoding. In some embodiments as used herein, intended actions refer to actions that the human subject intends to perform. In some embodiments, the first set of intended actions include an intended motor action, an intended cursor action, or an intended verbal action (e.g., speech action) for controlling the computing device. In some embodiments, the first set of intended actions corresponds to a first set of action types.

In some embodiments, the computing device, while operating in the first mode, controls (808) one or more interface devices that are communicatively connected with the computing device according to the first set of intended actions. The one or more interface devices include at least one of: a pointing device (a mouse 174, touchpad 175, or cursor), a keyboard 176, a display device (e.g., display 170 or external display device 173), a robotic device, and an audio output device (e.g., for speaking/verbalizing the intended action)

The computing device receives (810) an instruction to operate the computing device in a second mode (e.g., safe mode 193), different from the first mode.

In some embodiments, the instruction to operate the computing device in a second mode is generated by the computing device.

In some embodiments, entry into safe mode is based on one or more measured technical characteristics of the neural-signal acquisition and decoding pipeline. As one example of the computing device generating the instruction to operate the computing device in a second mode, in some embodiments, the computing device, while receiving the set of signals from the brain of the human subject in the first mode, determines (812) a quality of the set of signals, as illustrated in step 604 in Figure 6A. The quality of the signals can be determined according to a quality metric such as a signal-to-noise ratio (SNR) of the acquired neural signals, a signal (e.g., data) packet loss metric associated with transmission of neural data from the implant device to the wearable device or from the wearable device to the computing device, a latency metric (e.g., time it takes for signal to travel from implant device to wearable device, or from wearable device to the computing device), a jitter metric, an interference metric associated with interference with other devices, a metric associated with spurious emissions, a rate of user corrections to decoded output, a frequency of deletion or undo operations, and/or a confidence measure from a machine learning model (e.g., signal processing module 446, interpretation module 448, and/or data processing models 468/568). In some embodiments, the computing device determines that safe mode criteria are satisfied when one or more such metrics cross respective thresholds, or when a temporal trend of the metrics indicates persistent degradation over a time interval. For example, in some embodiments, the computing device, in accordance with a determination that the quality of the set of signals satisfies a threshold value (e.g., is less than or equal to a threshold value) (e.g., as indicated in step 606 in Figure 6A), generates the instruction to operate the computing device in the second mode and executes the instruction by switching from the first mode of operation to the second mode of operation (e.g., step 610, Enter safe mode).

As another example of the computing device generating the instruction to operate the computing device in a second mode, in some embodiments, for a respective determined intended action (e.g., intended action A 622, intended action B 624, and intended action N 626), the computing device determines (814) a respective confidence score (e.g., confidence scores 623, 625, or 627) corresponding to the respective determined intended action. In accordance with a determination that the respective confidence score does not satisfy a threshold confidence value (e.g., confidence score not met, as illustrated in steps 629, 631, and 633), the computing device generates the instruction to operate the computing device in the second mode and executes the instruction by switching from the first mode of operation to the second mode of operation (e.g., step 635: enter safe mode).

In some embodiments, the instruction to operate the computing device in the second mode is received (816) as a signal from the brain of the human subject. For example, in some embodiments, the human subject can transmit, via brain signals 602, a user override instruction (e.g., user override instruction 612, 640, 642, or 644) to operate the computing device in the second mode.

The computing device, in accordance with receiving the instruction, operates (818) the computing device in the second mode. The second mode limits operations of the computing device to a subset of operations, less than all of a plurality of operations that the computing device is configured to perform in the first mode. In some embodiments, the second mode corresponds to a safe mode of operation (e.g., safe mode 193). As described with reference to Figure 1B, in some embodiments the safe mode 193 is a fallback mode for when the high performance mode 191 is not available. In some embodiments, operating in safe mode includes reconfiguring the interpretation module (e.g., interpretation module 448 or 458) to decode a reduced action space selected for current reliability. For example, the computing device can reduce a first action space associated with the high performance operating mode 191, including speech decoding, text entry, application navigation, or control of external devices, to a second action space associated with safe mode 193, including a smaller number of cursor-direction commands, a binary selection command, a unary selection command, or a limited set of high-priority commands. In some embodiments, the computing device selects the second action space according to which control modality is currently decoded with higher reliability for the human subject, such as directional cursor movement, a predefined gesture, a binary select/no-select action, or another reduced-control modality. In some embodiments, the safe mode user interface is generated as a modal interface that captures command execution by the computing device while safe mode is active, thereby preventing lower-confidence neural decoding from being applied to unrestricted third-party software.

In some embodiments, operating the computing device in the second mode includes deactivating by the computing device (e.g., automatically, without user intervention) a speech decoding functionality of the computing device.

In some embodiments, operating the computing device in the second mode includes deactivating by the computing device (e.g., automatically, without user intervention) an active third-party application (e.g., a third-party application that is currently open and in use in the computing device, or actively performing a task in the foreground of the computing device). In some embodiments, operating the computing device in the second mode includes deactivating by the computing device (e.g., automatically, without user intervention) all active third-party applications.

In some embodiments, operating the computing device in the second mode includes controlling by the computing device (e.g., automatically, without user intervention) movement of a cursor (e.g., cursor 730) that is communicatively connected to the computing device to a set of predefined movements. For example, the set of predefined movements includes only movement in four directions (e.g., up, down, left, and right directions).

In some embodiments, the computing device, while operating in the second mode, determines (820) a second set (e.g., one or more) of intended actions of the human subject based on decoding the set of signals. The second set of intended actions is a subset of or different from the first set of intended actions.

In some embodiments, the second set of intended actions corresponds to a second set of action types that are different from action types corresponding to the first set of intended actions.

As disclosed herein, the second mode (e.g., safe mode) provides limited functioning of the computing device. In some embodiments, when the computing device operates in the second mode, the computing device takes the same set of brain signals and tries to do less with it. As one example, while operating in the first mode, the computing device may be configured to decode the received brain signals and determine intended actions such as (i) the user is trying to move a cursor or (ii) the user is trying to speak certain words, including deciphering the words, and outputs actions corresponding to the determined intended actions; While operating in the second mode, the computer device takes the same brain signals and performs a rudimentary set of operations involving cursor control (e.g., where the only possible outputs are cursor movement in an up, down, left, or right direction in the user interface). As another example, while operating in the first mode, the computing device is configured to interpret fifty (50) actions whereas in while operating in the second mode, the computing device is configured to interpret five (5) actions.

In some embodiments, the set of signals includes an attempted speech signal (e.g., a signal associated with an attempt by the human subject to speak). In some embodiments, while operating in the first mode, the machine learning algorithm is configured to decode the attempted speech signal by identifying one or more words from a predefined corpus of words and construct one or more sentences from the one or more words. For example, the predefined corpus of words can include a collection of at least 2000 words, 5000 words, 10,000 words or 20,000 words. In some embodiments, while operating in the second mode, the machine learning algorithm is configured to decode the attempted speech signal by classifying the attempted speech signal into a first utterance of a predefined set of utterances. For example, an utterance can be a word or a phrase. In some embodiments, each utterance in the predefined set of utterances corresponds to a respective word, and a total number (e.g., total count) of utterances in the predefined set of utterances is less than a total number (e.g., total count) of words in the predefined corpus of words.

With continued reference to Figure 8, in some embodiments, the computing device, when operating the computing device in the second mode, generates (822) and displays a user interface (e.g., safe mode user interface 710) that includes a set of user-selectable input controls (e.g., menu options). The set of user-selectable input controls corresponds to the subset of operations that the computing device is configured to perform in the second mode. In some embodiments, each input control corresponds to a respective operation that the computing device is configured to perform in the second mode. For example, as illustrated in Figure 7A, the set of input controls is displayed in respective regions of the safe mode UI 710. In some embodiments, the respective regions include a region 718 that, when selected (e.g., when the cursor 730 moves to that region), causes the computing device 140 to exit safe mode. In some embodiments, the respective regions include a region 720 that, when selected (e.g., when the cursor 730 moves to that region), causes the computing device to trigger a call for help. In some embodiments, the respective regions include one or more regions 722 (e.g., region 722-1 and region 722-2) corresponding to menu or action items.

In some embodiments, the user interface includes a first region (e.g., Home region 712) corresponding to an initial position of a cursor (e.g., 730); a second region (e.g., reinforcement learning region 714) adjacent to the first region; and a third region (e.g., region 718, region 720, region 722-1, and/or region 722-2) for displaying the set of user-selectable input controls (e.g., user-selectable icons, user-selectable options, user-selectable affordances). In some embodiments, each user-selectable input control of the set of user-selectable input controls is displayed as a distinct menu item in the third region of the user interface. In some embodiments, as illustrated in Figures 7A to 7E, the first, second, and third regions are non-overlapping regions. In some embodiments, the second region (e.g., reinforcement learning region 714) is contiguous to and surrounds the first region (e.g., Home region 712), as illustrated in Figures 7A to 7E.

In some embodiments, the user interface includes a fourth region (e.g., context region 716) positioned between the second region (e.g., reinforcement learning region 714) and the third region (e.g., region 718, region 720, region 722-1, and/or region 722-2). In some embodiments, the computing device, in accordance with a determination that a cursor is positioned within the fourth region (context region 716), displays information (e.g., guidance) about one or more actions associated with a respective user-selectable input control of the set of user-selectable input controls that are displayed in the third region. In some embodiments, the information about the one or more actions is displayed in a status bar 724 of the user interface, as illustrated in Figures 7C and 7D. In some embodiments, the information about the one or more actions is displayed as a tooltip 734 (e.g., pop-up window), as illustrated in Figure 7D.

In some embodiments, at least a subset of the user-selectable input controls is displayed as a carousel menu (e.g., carousel menu 740, Figure 7F) in the third region (e.g., region 722) of the user interface. The carousel menu includes a plurality of menu items (e.g., menu or action items 742-1, 742-2, and 742-3) and each menu item is a user-selectable input control.

In some embodiments, selection of a first menu item on the carousel menu includes, while operating in the second mode, decoding the set of signals and determining, based on the decoding, an intended action of the human subject to control the computing device to select a first user-selectable control corresponding to the first menu item. In some embodiments, the set of signals includes a signal associated with an attempt by the human subject to speak; or a signal associated with an attempt by the human subject to move a cursor; and/or a signal associated with an attempt by the human subject to perform a selection operation (e.g., a binary toggle or a click operation) with the cursor.

In some embodiments, the computing device limits (824) output of the intended actions to a cursor control when operating in the second mode.

In some embodiments, movement of the cursor to a particular region of the user interface corresponds to a respective intended action. In some embodiments, movement of a cursor by the computing device (without user interaction with the cursor) into a respective menu item indicates selection of an operation of the computing device corresponding to the respective menu item (e.g., depth-to-select, without clicking on the menu item).

In some embodiments, the computing device detects a cursor movement from the first region (e.g., Home region 712) to the second region (e.g., reinforcement learning region 714). In some embodiments, the computing device, in response to detecting the cursor movement, in accordance with a determination that the cursor movement from the first region to the second region (where the cursor movement that is generated by the computing device according to the received user's brain signals, without user interaction with the cursor) is followed by subsequent cursor movement from the second region toward the third region (where the subsequent cursor movement is generated/controlled by the computing device according to the received user's brain signals, without user interaction with the cursor), updates respective values (e.g., weights 572) of a set of parameters (e.g., model parameters) of a machine learning model (e.g., machine learning models 620, data processing models 480, or data processing models 568) for predicting cursor control from an initial set of values to a first set of values. In some embodiments, the computing device, in accordance with a determination that the cursor movement from the first region (e.g., Home region 712) to the second region e.g., reinforcement learning region 714) (where the cursor movement that is generated/controlled by the computing device without user interaction with the cursor) is followed by (i) a change in direction of the cursor movement or (ii) a termination of the cursor movement (i.e., cursor stops moving) (where the cursor movement that is generated/controlled by the computing device according to the received user's brain signals, without user interaction with the cursor), updates the respective values of the set of parameters of the machine learning model for predicting cursor control from the initial set of values to a second set of values, different from the first set of values.

For example, as described with reference to Figures 7A to 7E, the second region corresponds to the reinforcement learning region 714 and is used by the computing device 140 for reinforcement learning. If the initial decoded signals (decoded by the computing device) indicate that the user intended for the cursor to be moved from the first region to the second region, and subsequent decoded signals indicate that the user decided to change the direction of cursor movement or decided to stop cursor movement, the computing device can infer negative feedback for the selected label ("up," "down," etc). On the other hand, if the decoded signals indicate that the user intended to move the cursor into the second region and then continue to cause the cursor to be moved into the third region, the computing device can infer positive feedback for the selected label. Accordingly, interaction traces generated during safe mode are used to update one or more model parameters of the interpretation module 448/548 or an associated training module. For example, when the cursor is moved from the home region into a reinforcement-learning region and the subsequent neural input continues in the same direction toward a corresponding selectable region, the computing device treats the trajectory as positive reinforcement for the decoded direction. When the cursor is moved from the home region into the reinforcement-learning region and subsequent neural input causes a change in direction, a cessation of movement, or a return toward the home region, the computing device treats the trajectory as negative reinforcement for the decoded direction. In this way, the computing device uses user interaction with the safe mode interface to retrain or recalibrate decoding for the particular human subject, thereby improving later machine control without requiring a separate dedicated training session. In some embodiments, the computing device exits safe mode automatically after the updated decoding satisfies one or more recovery criteria, or transitions the human subject to a calibration mode for additional model refinement.

In some embodiments, the first mode corresponds to a normal mode of operation (e.g., high performance mode 191), and the second mode corresponds to a safe mode of operation (e.g., safe mode 193).

In some embodiments, the computing device generates and displays a first user interface (e.g., user interface 442) while operating in the first mode. In some embodiments, the computing device generates and displays a second user interface (e.g., safe mode user interface 710) while operating in the second mode. The first user interface and the second user interface are distinct user interfaces.

The methods disclosed herein comprise one or more steps or actions for achieving the described method. The method steps and/or actions may be interchanged with one another without departing from the scope of the claims. In other words, unless a specific order of steps or actions is required for proper operation of the method that is being described, the order and/or use of specific steps and/or actions may be modified without departing from the scope of the claims.

Turning now to some example embodiments:
(A1) In accordance with some embodiments, a method is performed at a computing device that includes one or more processors and memory. The method comprises, (i) while operating in a first mode, decoding a set of signals that are received from a brain of a human subject; (ii) determining a first set of intended actions of the human subject based on the decoding, the first set of intended actions including an intended motor action, an intended cursor action, or an intended verbal action for controlling the computing device; (iii) receiving a first instruction to operate the computing device in a second mode, different from the first mode; and (iv) in accordance with receiving the first instruction, operating the computing device in the second mode, wherein the second mode limits operations of the computing device to a subset of operations, less than all of a plurality of operations that the computing device is configured to perform in the first mode.
(A2) In some embodiments of A1, the set of signals comprises one or more of: (i) a signal associated with an attempt by the human subject to speak; (ii) a signal associated with an attempt by the human subject to move a body part of the human subject; (iii) a signal associated with an attempt by the human subject to control a facial expression of the human subject; and (iv) a signal associated with an attempt by the human subject to control an external device.
(A3) In some embodiments of any of A1-A2, while operating in the first mode, decoding the set of signals that are received from the brain of a human subject includes: (i) inputting the set of signals into a machine learning algorithm; and (ii) obtaining one or more intended actions from the machine learning algorithm.
(A4) In some embodiments of A3, the machine learning algorithm is associated with one or more machine learning models that are trained according to data from the human subject.
(A5) In some embodiments of any of A3-A4, the machine learning algorithm comprises a plurality of decoder models that are configured to output representations corresponding to a plurality of output control modalities.
(A6) In some embodiments of any of A3-A5, the set of signals includes an attempted speech signal, and the machine learning algorithm is configured to: (i) decode the attempted speech signal by identifying one or more words from a predefined corpus of words and construct one or more sentences from the one or more words while operating in the first mode; and (ii) decode the attempted speech signal by classifying the attempted speech signal into a first utterance of a predefined set of utterances while operating in the second mode.
(A7) In some embodiments of A6, wherein each utterance in the predefined set of utterances corresponds to a respective word, and a total number of utterances in the predefined set of utterances is less than a total number of words in the predefined corpus of words.
(A8) In some embodiments of any of A1-A7, the method further comprises while operating in the second mode, determining a second set of intended actions of the human subject based on decoding the set of signals, wherein the second set of intended actions is a subset of or different from the first set of intended actions.
(A9) In some embodiments of any of A1-A8, operating the computing device in the second mode includes generating and displaying a user interface that includes a set of user-selectable input controls, the set of user-selectable input controls corresponding to the subset of operations that the computing device is configured to perform in the second mode.
(A10) In some embodiments of A9, wherein movement of a cursor in a particular direction of the user interface corresponds to a respective intended action of the human subject.
(A11) In some embodiments of A9 or A10, wherein the user interface includes (i) a first region corresponding to an initial position of a cursor; (ii) a second region adjacent to the first region; and (iii) a third region for displaying the set of user-selectable input controls.
(A12) In some embodiments of A11, the second region is contiguous to and surrounds the first region.
(A13) In some embodiments of any of A11-A12, wherein movement of a cursor to a particular region of the user interface corresponds to a respective intended action.
(A14) In some embodiments of any of A11-A13, the method further comprises (a) detecting a cursor movement from the first region to the second region; and (b) in response to detecting the cursor movement: (b-1) in accordance with a determination that the cursor movement from the first region to the second region is followed by subsequent cursor movement from the second region toward the third region, updating respective values of a set of parameters of a machine learning model for predicting cursor control from an initial set of values to a first set of values; and (b-2) in accordance with a determination that the cursor movement from the first region to the second region is followed by (i) a change in direction of the cursor movement or (ii) a termination of the cursor movement, updating the respective values of the set of parameters of the machine learning model for predicting cursor control from the initial set of values to a second set of values, different from the first set of values.
(A15) In some embodiments of any of A11-A14, the user interface includes a fourth region positioned between the second region and the third region; and the method further comprises, in accordance with a determination that a cursor is positioned within the fourth region, displaying information about one or more actions associated with a respective user-selectable input control of the set of user-selectable input controls that are displayed in the third region.
(A16) In some embodiments of any of A11-A15, wherein each user-selectable input control of the set of user-selectable input controls is displayed as a distinct menu item in the third region of the user interface.
(A17) In some embodiments of A16, wherein movement of a cursor by the computing device into a respective menu item indicates selection of an operation of the computing device corresponding to the respective menu item.
(A18) In some embodiments of any of A11-A17, wherein at least a subset of the user-selectable input controls is displayed as a carousel menu in the third region of the user interface, the carousel menu including a plurality of menu items, and each menu item is a user-selectable input control.
(A19) In some embodiments of A18, wherein selection of a first menu item on the carousel menu includes: (i) while operating in the second mode, decoding the set of signals; and (ii) determining, based on the decoding, an intended action of the human subject to control the computing device to select a first user-selectable control corresponding to the first menu item.
(A20) In some embodiments of A19, wherein the set of signals include one or more of: (i) a signal associated with an attempt by the human subject to speak; (ii) a signal associated with an attempt by the human subject to move a cursor; and (iii) a signal associated with an attempt by the human subject to perform a selection operation with the cursor.
(A21) In some embodiments of any of A1-A20, wherein operating the computing device in the second mode includes deactivating a speech decoding functionality of the computing device.
(A22) In some embodiments of any of A1-A21, wherein operating the computing device in the second mode includes controlling movement of a cursor that is communicatively connected to the computing device to a set of predefined movements.
(A23) In some embodiments of any of A1-A22, wherein operating the computing device in the second mode includes deactivating an active third-party application.
(A24) In some embodiments of any of A1-A23, wherein operating the computing device in the second mode includes deactivating all active third-party applications.
(A25) In some embodiments of any of A1-A24, wherein determining the first set of intended actions of the human subject based on the decoding includes, for a respective determined intended action: (i) determining a respective confidence score corresponding to the respective determined intended action; and (ii) in accordance with a determination that the respective confidence score does not satisfy a threshold confidence value, generating the first instruction to operate the computing device in the second mode and executing the first instruction by switching from the first mode of operation to the second mode of operation.
(A26) In some embodiments of any of A1-A25, wherein the first instruction to operate the computing device in the second mode is received as a signal from the brain of the human subject.
(A27) In some embodiments of any of A1-A26, the method further comprises: (i) while receiving the set of signals from the brain of the human subject in the first mode, determining a quality of the set of signals; and (ii) in accordance with a determination that the quality of the set of signals satisfies a threshold value, generating the first instruction to operate the computing device in the second mode.
(A28) In some embodiments of any of A1-A27, the method further comprises: (i) generating and displaying a first user interface while operating in the first mode; and (ii) generating and displaying a second user interface while operating in the second mode, wherein the first user interface and the second user interface are distinct user interfaces.
(A29) In some embodiments of any of A1-A28, wherein the first mode corresponds to a normal mode of operation; and the second mode corresponds to a safe mode of operation.
(A30) In some embodiments of any of A1-A29, the method further comprises while operating in the first mode, controlling one or more interface devices that are communicatively connected with the computing device according to the first set of intended actions, the one or more interface devices including at least one of: a pointing device, a keyboard, a display device, a robotic device, and an audio output device.
(A31) In some embodiments of any of A1-A30, wherein operating the computing device in the second mode of operation includes limiting output of the intended actions to a cursor control.
(B1) In accordance with some embodiments, a computing device comprises one or more processors and memory coupled to the one or more processors, the memory storing one or more programs configured for execution by the one or more processors, the one or more programs including instructions that, when executed by the one or more processors, cause the computing device to perform the method of any of any of A1-A31.
(C1) In accordance with some embodiments, a non-transitory computer-readable storage medium stores one or more programs, the one or more programs comprising instructions, which when executed by a computing device that includes one or more processors and memory, cause the computing device to perform the method of any of A1-A31

It will be understood that, although the terms "first," "second," etc. may be used herein to describe various elements, these elements should not be limited by these terms. These terms are only used to distinguish one element from another. The terminology used herein is for the purpose of describing particular embodiments only and is not intended to be limiting of the claims. As used in the description of the embodiments and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "plurality" denotes two or more. For example, a plurality of components indicates two or more components. The term "determining" encompasses a wide variety of actions and, therefore, "determining" can include calculating, computing, processing, deriving, investigating, looking up (e.g., looking up in a table, a database or another data structure), ascertaining and the like. Also, "determining" can include receiving (e.g., receiving information), accessing (e.g., accessing data in a memory) and the like. Also, "determining" can include resolving, selecting, choosing, establishing and the like.

The phrase "based on" does not mean "based only on," unless expressly specified otherwise. In other words, the phrase "based on" describes both "based only on" and "based at least on."

As used herein, the term "exemplary" means "serving as an example, instance, or illustration," and does not necessarily indicate any preference or superiority of the example over any other configurations or embodiments.

As used herein, the term "and/or" encompasses any combination of listed elements. For example, "A, B, and/or C" entails each of the following possibilities: A only, B only, C only, A and B without C, A and C without B, B and C without A, and a combination of A, B, and C.

The terminology used in the description of the invention herein is for the purpose of describing particular embodiments only and is not intended to be limiting of the invention. As used in the description of the invention and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof.

The foregoing description, for the purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the invention and its practical applications, to thereby enable others skilled in the art to best utilize the invention and various embodiments with various modifications as are suited to the particular use contemplated.

## Claims

1. A method of operating in safe mode, performed at a computing device that includes one or more processors, and memory, the method comprising:
while operating in a first mode, decoding a set of signals that are received from a brain of a human subject;
determining a first set of intended actions of the human subject based on the decoding, the first set of intended actions including an intended motor action, an intended cursor action, or an intended verbal action for controlling the computing device;
receiving a first instruction to operate the computing device in a second mode, different from the first mode; and
in accordance with receiving the first instruction, operating the computing device in the second mode, wherein the second mode limits operations of the computing device to a subset of operations, less than all of a plurality of operations that the computing device is configured to perform in the first mode.

2. The method of any preceding claim, wherein the set of signals comprises one or more of:
a signal associated with an attempt by the human subject to speak;
a signal associated with an attempt by the human subject to move a body part of the human subject;
a signal associated with an attempt by the human subject to control a facial expression of the human subject; and
a signal associated with an attempt by the human subject to control an external device.

3. The method of any preceding claim, wherein while operating in the first mode, decoding the set of signals that are received from the brain of a human subject includes:
inputting the set of signals into a machine learning algorithm; and
obtaining one or more intended actions from the machine learning algorithm.

4. The method of claim 3, wherein the set of signals includes an attempted speech signal, and the machine learning algorithm is configured to:
decode the attempted speech signal by identifying one or more words from a predefined corpus of words and construct one or more sentences from the one or more words while operating in the first mode; and
decode the attempted speech signal by classifying the attempted speech signal into a first utterance of a predefined set of utterances while operating in the second mode.

5. The method of any preceding claim, including:
while operating in the second mode, determining a second set of intended actions of the human subject based on decoding the set of signals, wherein the second set of intended actions is a subset of or different from the first set of intended actions.

6. The method of any preceding claim, wherein operating the computing device in the second mode includes:
generating and displaying a user interface that includes a set of user-selectable input controls, the set of user-selectable input controls corresponding to the subset of operations that the computing device is configured to perform in the second mode.

7. The method of claim 6, wherein movement of a cursor in a particular direction of the user interface corresponds to a respective intended action of the human subject.

8. The method of claim 6 or claim 7, wherein the user interface includes:
a first region corresponding to an initial position of a cursor;
a second region contiguous to and surrounding the first region; and
a third region for displaying the set of user-selectable input controls;
wherein movement of a cursor to a particular region of the user interface corresponds to a respective intended action.

9. The method of claim 8, including:
detecting a cursor movement from the first region to the second region; and
in response to detecting the cursor movement:
in accordance with a determination that the cursor movement from the first region to the second region is followed by subsequent cursor movement from the second region toward the third region, updating respective values of a set of parameters of a machine learning model for predicting cursor control from an initial set of values to a first set of values; and
in accordance with a determination that the cursor movement from the first region to the second region is followed by (i) a change in direction of the cursor movement or (ii) a termination of the cursor movement, updating the respective values of the set of parameters of the machine learning model for predicting cursor control from the initial set of values to a second set of values, different from the first set of values.

10. The method of any preceding claim, wherein operating the computing device in the second mode includes one or more of:
deactivating a speech decoding functionality of the computing device;
limiting movement control of a cursor that is communicatively connected to the computing device to a set of predefined movements;
deactivating an active third-party application;
deactivating all active third-party applications; and
limiting output of the intended actions to a cursor control.

11. The method of any preceding claim, wherein determining the first set of intended actions of the human subject based on the decoding includes:
for a respective determined intended action:
determining a respective confidence score corresponding to the respective determined intended action; and
in accordance with a determination that the respective confidence score does not satisfy a threshold confidence value, generating the first instruction to operate the computing device in the second mode and executing the first instruction by switching from the first mode of operation to the second mode of operation.

12. The method of any preceding claim, including:
while receiving the set of signals from the brain of the human subject in the first mode, determining a quality of the set of signals; and
in accordance with a determination that the quality of the set of signals satisfies a threshold value, generating the first instruction to operate the computing device in the second mode.

13. The method of any preceding claim, including:
generating and displaying a first user interface while operating in the first mode; and
generating and displaying a second user interface while operating in the second mode, wherein the first user interface and the second user interface are distinct user interfaces.

14. A computing device, comprising:
one or more processors; and
memory coupled to the one or more processors, the memory storing one or more programs configured for execution by the one or more processors, the one or more programs including instructions for performing or causing performance of the method of any one of claims 1-13.

15. A computer-readable storage medium including one or more programs comprising instructions, which when executed by a computing device that includes one or more processors and memory, cause the computing device to perform the method of any one of claims 1-13.
